# DEMANDE DE BREVET EUROPEEN

(11) **EP 2 853 534 A1**
(43) Date de publication de la demande: **01.04.2015**
(21) Numéro de dépôt: 14192364.9
(22) Date de dépôt: 29.07.2009
(51) Int. Cl.: C07D 487/18, A61K 31/437, A61P 31/04

(54) **Nouveaux composés hétérocycliques azotes, leur préparation et leur utilisation comme médicaments antibactériens**

(30) Priorité: 10.10.2008 FR 0805602
(62) Demande divisionnaire de: 09786084.5
(71) Demandeur: ASTRA ZENECA HOLDING FRANCE, 92500 Rueil Malmaison (FR)
(72) Inventeur: Ledoussal, Benoît, 22450 Pommerit Jaudy (FR); Gourdel, Marie-Edith, 77176 Savigny le Temple (FR); Renaud, Emilie, 75013 Paris (FR); Pierres, Camille, 75013 Paris (FR); Kebsi, Adel, 94320 Thiais (FR)
(74) Mandataire: Hirsch & Associés

(57) **Abrégé**

**NOUVEAUX COMPOSES HETEROCYCLIQUES AZOTES, LEUR PREPARATION ET LEUR UTILISATION COMME MEDICAMENTS ANTIBACTERIENS**

L'invention concerne des composés hétérocycliques azotés de formule (I) : 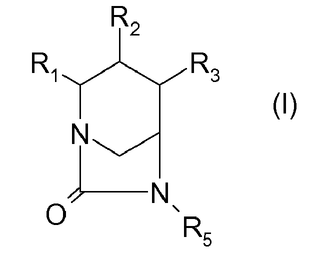 dans laquelle :
R₁ représente -(CH₂)ₘ-NH₂, -(CH₂)ₘ-NH(C₁-C₆)alc, -(CH₂)ₘ-N(C₁-C₆)alc₂, -(CH₂)ₘ-NH-C(NH)NH₂ ou -(CH₂)ₘ-NH-CH=NH, m est égal à 1 ou 2;
R₂ et R₃ forment ensemble un hétérocycle azoté à caractère aromatique à 5 sommets renfermant 1, 2 ou 3 atomes d'azote, substitué sur un atome d'azote par R₄;
R₄ représente hydrogène, (C₁-C₆)alc ou une chaîne de formule :

-(A)ₙ-(NH)ₒ-(CH₂)ₚ-(CHR')_{q}R" ;

A représente C=O, C=NH ou SO₂;
R' représente hydrogène ou carboxy;
R" représente hydrogène, NH₂, NH(C₁-C₆)alc, N(C₁-C₆)alC₂, CONH₂, CONH(C₁-C₆)alc, CON(C₁-C₆)alc₂, ou un hétérocycle saturé à 5 ou 6 sommets renfermant 1 ou 2 azotes et le cas échéant un oxygène ou un soufre, fixé à la chaîne par un azote ou par un carbone et éventuellement substitué par (C₁-C₆)alc ;
n, o et q représentent 0 ou 1 et p représente un entier de 0 à 4 ;
R₅ représente OSO₃H, OCFCO₂H ou OCF₂CO₂H;
- R₁ étant différent -(CH₂)ₘ-NH₂, -(CH₂)ₘ-NH(C₁-C₆)alc ou -(CH₂)ₘ-N(C₁-C₆)alc₂ lorsque R₄ est hydrogène, -(C₁-C₆)alc, -(C=O)ₙ-(CH₂)₍₀₋₅₎-NH₂, -(C=O)ₙ-(CH₂)₍₀₋₅₎-NH(C₁-C₆)alc ou -(C=O)ₙ-(CH₂)₍₀₋₅₎-N(C₁-C₆)alc₂ et R₅ est un groupe OSO₃H, ou lorsque R₄ a l'ensemble des valeurs de R" ci-dessus à l'exception d'hétérocycle,
- et n, o, p et q ne pouvant tous être 0 sauf lorsque R" est hydrogène ou CONH₂, CONH(C₁-C₆)alc, CON(C₁-C₆)alc₂ ou hétérocycle ;

sous forme libre et sous forme de zwitterions et de sels avec les bases et les acides minéraux ou organiques pharmaceutiquement acceptables, leur préparation et leur utilisation comme médicaments antibactériens.

## Description

L'invention concerne des composés hétérocycliques azotés à titre de médicaments antibactériens.

Les demandes WO 02/010172, 02/100860, 04/022563 et 04/052891 décrivent des composés polycycliques utiles dans la lutte contre les bactéries pathogènes.

La demanderesse a découvert de nouveaux composés apparentés, possédant des remarquables propriétés antibactériennes tout à fait inattendues. Ces composés possèdent plus particulièrement une excellente activité sur *Pseudomonas aeruginosa,* une souche bactérienne fréquemment rencontrée dans les infections nocosomiales ainsi que chez les patients souffrant de mucoviscidose.

Cette activité intéressante et inattendue n'est pas présente dans les composés des demandes citées plus haut. Elle est illustrée plus loin dans la partie expérimentale.

Par ailleurs, les composés de l'invention se sont montrés actifs sur des modèles d'infection animale, y compris sur des souches habituellement résistantes aux antibiotiques communément utilisés. Les composés de l'invention sont capables de contrecarrer les principaux mécanismes de résistance des bactéries que sont les β-lactamases, les pompes à efflux et les mutations des porines.

Les composés de l'invention répondent à la formule (I), sous leurs formes isomères ou diastéroisomères possibles, ou de mélanges: 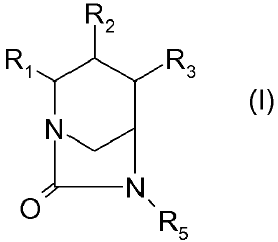 dans laquelle :
R₁ représente un radical -(CH₂)ₘ-NH₂,
   -(CH₂)ₘ-NH(C₁-C₆)alc, -(CH₂)ₘ-N(C₁-C₆)alc₂, -(CH₂)ₘ-NH-C(NH)NH₂ ou -(CH₂)ₘ-NH-CH=NH, dans lequel m est égal à 1 ou 2;
R₂ et R₃ forment ensemble un hétérocycle azoté à caractère aromatique à 5 sommets renfermant 1, 2 ou 3 atomes d'azote, substitué sur un atome d'azote par R₄;
R₄ représente un atome d'hydrogène, un radical (C₁-C₆)alc ou une chaîne de formule :

   -(A)ₙ-(NH)ₒ-(CH₂)ₚ-(CHR')_{q}R''
A représente un groupe C=O, C=NH ou SO₂;
R' représente un atome d'hydrogène ou un groupe carboxy;
R" représente un atome d'hydrogène ou un groupe NH₂, NH(C₁-C₆)alc, N(C₁-C₆)alc₂, CONH₂, CONH(C₁-C₆)alc, CON(C₁-C₆)alc₂, ou un hétérocycle saturé à 5 ou 6 sommets renfermant 1 ou 2 atomes d'azote et, le cas échéant un autre hétéroatome choisi parmi l'oxygène et le soufre, fixé à la chaîne par un atome d'azote ou par un atome de carbone et éventuellement substitué par un radical (C₁-C₆)alc ;
n, o et q représentent 0 ou 1 et p représente un entier de 0 à 4 ;
R₅ représente un groupe OSO₃H ou OCFHCO₂H ou OCF₂CO₂H;
étant entendu que :
   - R₁ est différent de -(CH₂)ₘ-NH₂, -(CH₂)ₘ-NH(C₁-C₆)alc ou -(CH₂)ₘ-N(C₁-C₆)alc₂ lorsque R₄ est hydrogène, -(C₁-C₆)alc, -(C=O)ₙ-(CH₂)₍₀₋₅₎-NH₂, -(C=O)ₙ-(CH₂)₍₀₋₅₎-NH(C₁-C₆)alc ou - (C=O)ₙ-(CH₂)₍₀₋₅₎-N(C₁-C₆)alc₂ et R₅ est un groupe OSO₃H, ou lorsque R₄ a l'ensemble des valeurs de R" ci-dessus à l'exception d'hétérocycle tel que défini plus haut,
   - et n, o, p et q ne peuvent être tous égaux à 0 sauf lorsque R" est hydrogène ou un groupe CONH₂, CONH(C₁-C₆)alc, CON(C₁-C₆)alc₂, ou un hétérocycle ;
sous forme libre et sous forme de zwitterions et de sels avec les bases et les acides minéraux ou organiques pharmaceutiquement acceptables.

Par radical (C₁-C₆) alkyle, on entend notamment le radical méthyle, éthyle, propyle, isopropyle, ainsi que butyle, pentyle ou hexyle linéaire ou ramifié.

Par hétérocycle à caractère aromatique à 5 sommets renfermant 1, 2 ou 3 atomes d'azote, on entend ceux choisis dans la liste qui suit, les deux liaisons symbolisant la jonction avec le cycle azoté formé par R₂ et R₃:

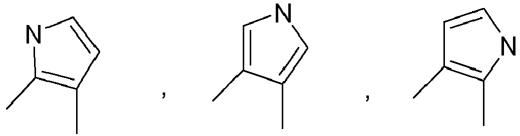

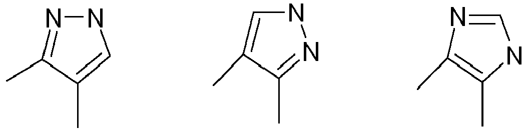

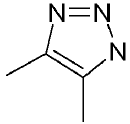

Par hétérocycle saturé à 5 ou 6 sommets renfermant 1 ou 2 atomes d'azote et, le cas échéant, un atome d'oxygène ou de soufre, on entend en particulier un cycle à 5 sommets de type pyrrolidine, imidazolidine ou pyrazolidine, ou un cycle à 6 sommets de type pipéridine, pipérazine, morpholine ou thiomorpholine, l'hétérocycle étant relié à la chaîne par un atome d'azote ou par un atome de carbone.

Parmi les sels d'acides des produits de formule (I), on peut citer entre autres, ceux formés avec les acides minéraux tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl)aminométhane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkylphosphonium, les arylphosphoniums, les alkylarylphosphonium, les alkénylarylphosphonium ou les sels d'ammoniums quaternaires tels que le sel de tétra n-butylammo nium.

Les atomes de carbone asymétriques contenus dans les composés de formule (I) peuvent indépendamment les uns des autres présenter la configuration R, S ou RS et les composés de formule (I) se présentent donc sous la forme d'énantiomères purs ou de diastéréoisomères purs ou sous la forme d'un mélange d'énantiomères notamment de racémates, ou de mélanges de diastéréoisomères.

En outre, le substituant R₁ d'une part et la chaîne
-C(O)-NR₅- d'autre part pouvant être en position cis et/ou trans par rapport au cycle sur lequel ils sont fixés, les composés de formule (I) se présentent sous la forme d'isomères cis ou d'isomères trans ou de mélanges.

Parmi les composés de formule (I) telle que définie plus haut, l'invention a notamment pour objet les composés dans lesquels R₂ et R₃ forment ensemble un hétérocycle pyrazolyle.

Parmi les composés de formule (I) telle que définie précédemment, l'invention a notamment pour objet ceux dans lesquels R₁ représente un radical -(CH₂)ₘ-NH₂, ainsi que ceux dans lesquels R₁ représente un radical -(CH₂)ₘ-NH-C(NH)NH₂, m dans l'un et l'autre cas étant égal à 1.

Parmi les composés de formule (I), l'invention a aussi notamment pour objet ceux dans lesquels R₄ représente une chaîne de formule -(A)ₙ-(NH)ₒ-(CH₂)ₚ-(CHR')_{q}R" telle que définie précédemment, et tout particulièrement ceux dans lesquels R₄ représente une chaîne de formule -C(O)-NH-(CH₂)ₚ-(CHR')_{q}R'' dans laquelle R', R", p et q sont tels que définis plus haut.

Parmi les composés de formule (I), l'invention a encore notamment pour objet ceux dans lesquels R₄ représente un atome d'hydrogène ou un radical (C₁-C₆)alc et R₁ représente un radical -(CH₂)ₘ-NH-C(NH)NH₂ ou -(CH₂)ₘ-NH-CH=NH, dans lequel m est égal à 1.

Parmi les composés de formule (I), l'invention a tout particulièrement pour objet les composés décrits plus loin dans la partie expérimentale et notamment ceux dont les noms suivent:
- la trans 8-(aminométhyl)-2-(2-amino-éthyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-2-(4-pipérazine-1-carbonyl)-4,8-dihydro-5-(sulfooxy)-4,7-méthano-7H-pyrazolo[3,4-e] [1,3]diazépin-6(5H)-one,
- la trans 8-(aminométhyl)-4,8-dihydro-2-(2-diméthylamino-éthyl-carbamoyl)-5-(sulfooxy)-4,7-méthano-7H-pyrazolo[3,4-e] [1,3] diazépin-6(5H)-one,
- la trans 8-(aminométhyl)-2-(3-amino-propyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-2-(carbamoyl-méthyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-1-(carbanimidoyl)-4,8-dihydro-5-(sulfooxy)-4,7-méthano-7H-pyrazolo[3,4-e] [1,3] diazépin-6(5H)-one,
- la trans 8-(aminométhyl)-2-(carbanimidoyl)-4,8-dihydro-5-(sulfooxy)-4,7-méthano-7H-pyrazolo[3,4-e] [1,3] diazépin-6(5H)-one,
- la trans 8-(amino-méthyl)-4,8-dihydro-2-(pipéridine-4-carbonyl)-5-(sulfooxy)-4,7-méthano-7H-pyrazolo[3,4-e] [1,3] diazépin-6(5H)-one,
- la trans 8-(amino-méthyl)-2-(3-amino-3-carboxy-propyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-1-méthyl-5-(sulfooxy)-4,7-méthano-7H-pyrazolo[3,4-e] [1,3] diazépin-6(5H)-one,
- la trans 8-(guanidino-méthyl)-2-carbamoyl-4,8-dihydro-(sulfooxy)-4,7-méthano-7H-pyrazolo[3,4-e] [1,3] diazépin-6(5H)-one,
- la trans 8-(amino-méthyl)-4,8-dihydro-1-méthyl-5-(carboxy-difluoro-méthoxy)-4,7-méthano-7H-pyrazolo[3,4-e] [1,3] diazépin-6(5H)-one,
- la trans 8-(amino-méthyl)-2-(amino-carbamoyle)-4,8-dihydro-5-(sulfooxy)-4,7-méthano-7H-pyrazolo[3,4-e] [1,3] diazépin-6(5H)-one,
sous forme libre, de zwittterions et de sels avec les bases et les acides minéraux ou organiques pharmarceutiquement acceptables, et sous leurs formes isomères ou diastéroisomères possibles, ou de mélanges.

Un autre objet de l'invention est un procédé de préparation de composés de formule (I), caractérisé en ce que l'on traite un composé de formule (II) : 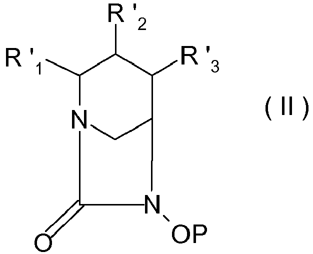 das laquelle R'₁ représente un radical R₁ dans lequel, le cas échéant, la ou les fonctions amino présentes sont protégées, R'₂ et R'₃ forment ensemble un hétérocycle azoté à caractère aromatique à 5 sommets renfermant 1, 2 ou 3 atomes d'azote et P représente un groupement protecteur du radical hydroxy, en présence d'une base, par le diphosgène, puis par une amine de formule (III) :

H-NH-(CH₂)ₚ-(CHR'ₐ)_{q}R"' (III)

dans laquelle R'ₐ et R"' représentent repectivement R' et R" dans lesquels, le cas échéant, les fonctions réactives carboxy et amino sont protégées, et p et q sont tels que définis plus haut, pour obtenir un composé de formule (IV) : 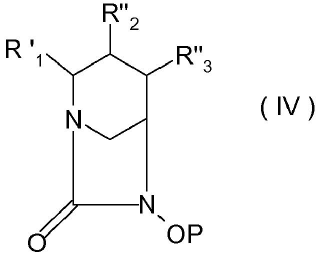 dans laquelle R'₁ et P sont tels que définis plus haut et R"₂ et R"₃ forment ensemble un hétérocycle azoté à caractère aromatique à 5 sommets renfermant 1, 2 ou 3 atomes d'azote, substitué sur un atome d'azote par une chaîne de formule -C(O)-NH-(CH₂)ₚ-(CHR'ₐ)_{q} R"' dans laquelle R'ₐ, R"', p et q sont tels que définis plus haut,
puis déprotège le radical hydroxy et soumet le composé obtenu à une réaction de sulfatation par action de SO₃ complexé, ou à l'action d'un réactif de formule Hal-CHF-CO₂alc ou de formule Hal-CF₂-CO₂alc, dans laquelle Hal représente un atome d'halogène différent du fluor et alc représente un radical alcoyle renfermant de 1 à 6 atomes de carbone, en présence d'une base, puis à une hydrolyse de l'ester d'alcoyle obtenu,
puis, le cas échéant, soumet le composé obtenu à une ou plusieurs des réactions suivantes, dans un ordre approprié :
- déprotection de la ou des fonctions aminées et, le cas échéant carboxy, présentes,
- salification,
- échange d'ions,
- dédoublement ou séparation de diastéréoisomères.

Un autre objet de l'invention est un procédé de préparation de composés de formule (I), caractérisé en ce que l'on traite un composé de formule (II) telle que définie plus haut, par une base, puis par un réactif de formule (V) :

Hal-SO₂-NH-(CH₂)ₚ-(CHR'ₐ)_{q}R"' (V)

dans laquelle Hal représente un atome d'halogène et R'ₐ, R"', p et q sont tels que définis plus haut, pour obtenir un composé de formule (IVa) : 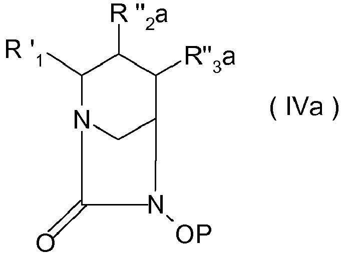 dans laquelle R'₁ et P sont tels que définis plus haut et R"₂ₐ et R"₃ₐ forment ensemble un hétérocycle azoté à caractère aromatique à 5 sommets renfermant 1, 2 ou 3 atomes d'azote, substitué sur un atome d'azote par une chaîne de formule -SO₂-NH-CH₂)ₚ-(CHR'ₐ)_{q} R"' dans laquelle R'ₐ, R"', p et q sont tels que définis plus haut,
puis déprotège le radical hydroxy et poursuit la synthèse comme décrit plus haut.

Un autre objet de l'invention est un procédé de préparation de composés de formule (I), caractérisé en ce que l'on traite un composé de formule (II) telle que définie plus haut, le cas échéant en présence d'une base, par un réactif de formule (VI) :

B-C(O)-(NH)ₒ-(CH₂)ₚ-(CHR'ₐ)_{q} R"' (VI)

dans laquelle B représente un radical OH ou un atome d'halogène et R'ₐ, R"', o, p et q sont tels que définis plus haut, pour obtenir un composé de formule (IVb) : 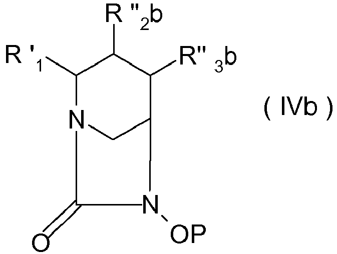 dans laquelle R'₁ et P sont tels que définis plus haut et R"_{2b} et R"_{3b} forment ensemble un hétérocycle azoté à caractère aromatique à 5 sommets renfermant 1, 2 ou 3 atomes d'azote, substitué sur un atome d'azote par une chaîne de formule -C(O)-(NH)ₒ-(CH₂)p-(CHR'ₐ)_{q} R"' dans laquelle R'ₐ, R"', o, p et q sont tels que définis plus haut, puis poursuit la synthèse comme décrit plus haut.

Un autre objet de l'invention est un procédé de préparation de composés de formule (I), caractérisé en ce que l'on traite un composé de formule (II) telle que définie plus haut, en présence d'une base, par un réactif de formule (VII) :

S=C(NHP')₂ (VII)

laquelle P' représente un groupement protecteur de la fonction amino, pour obtenir un composé de formule (IVc) : 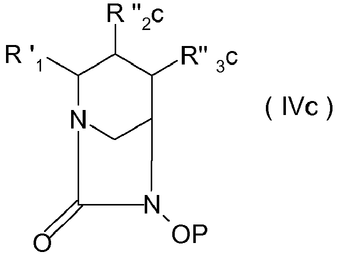 dans laquelle R'₁ et P sont tels que définis plus haut et R"_{2c} et R"_{3c} forment ensemble un hétérocycle azoté à caractère aromatique à 5 sommets renfermant 1, 2 ou 3 atomes d'azote, substitué sur un atome d'azote par une chaîne de formule -C(=NH)-NHP' dans laquelle P' est tel que défini plus haut,
puis poursuit la synthèse décrit comme plus haut.

Un autre objet de l'invention est un procédé de préparation de composés de formule (I), caractérisé en ce que l'on traite un composé de formule (II) telle que définie plus haut, par un réactif de formule (VIII) :

O=C=N-(CH₂)ₚ-(CHR'ₐ)_{q} R"' (VIII)

dans laquelle R'ₐ, R"', p et q sont tels que définis plus haut, pour obtenir un composé de formule (IVd) 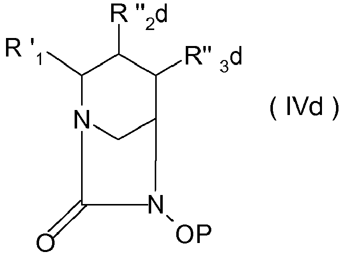 dans laquelle R'₁ et P sont tels que définis plus haut et R"_{2d} et R"_{3d} forment ensemble un hétérocycle azoté à caractère aromatique à 5 sommets renfermant 1, 2 ou 3 atomes d'azote, substitué sur un atome d'azote par une chaîne de formule -(CO)-NH-(CH₂)ₚ-(CHR'ₐ)_{q} R"' dans laquelle R'ₐ, R"', p et q sont tels que définis plus haut, puis poursuit la synthèse décrit comme plus haut.

Un composé de formule (I) dans laquelle R₄ représente un groupe CO-NH₂ ou CO-NH(C₁-C₆)alc peut encore être obtenu par action du composé de formule (II) telle que définie plus haut, avec le triméthylsilyl isocyanate ou avec un isocyanate de formule (C₁-C₆)alc-N=C=O, pour obtenir un composé correspondant de formule (IV), puis poursuit la synthèse comme décrit plus haut.

Un autre objet de l'invention est un procédé de préparation de composés de formule (I), caractérisé en ce que l'on traite un composé de formule (II') : 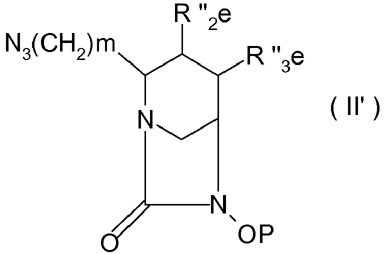 dans laquelle R"₂ₑ et R"₃ₑ forment ensemble un hétérocycle azoté à caractère aromatique à 5 sommets renfermant 1, 2 ou 3 atomes d'azote éventuellement substitué par un radical (C₁-C₆)alc et P est tel que défini plus haut, par un réactif de formule (IX) :

CH₃-S-C(=NP')NHP' (IX)

dans laquelle P' est tel que défini plus haut, pour obtenir un composé de formule (X) : 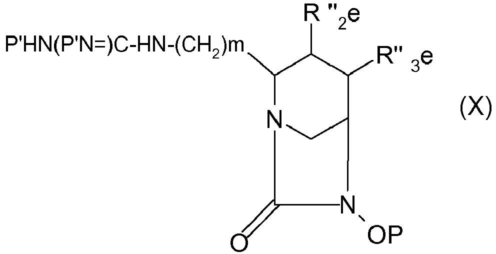 dans laquelle R"₂ₑ, R"₃ₑ, m, P et P' sont tels que définis plus haut,
puis poursuit la synthèse décrit comme plus haut.

La protection préalable de la fonction amine au niveau de R'₁ et dans les réactifs de formules III, V, VI, VII, VIII et IX est notamment effectuée sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényltertbutyl-silyle, ou encore de dérivés phénylsulfonylalkyle ou cyanoalkyle.

La déprotection peut être effectuée par différentes méthodes connues de l'homme du métier, selon la nature du groupement protecteur. Elle peut notamment être effectuée par action d'un acide, par exemple l'acide trifluoroacétique, le composé déprotégé étant alors obtenu sous forme de sel de l'acide. Elle peut encore être effectuée par hydrogénolyse ou à l'aide de complexes solubles du Palladium O ou par action du fluorure de tétrabutylammonium ou par réduction.Une illustration est fournie plus loin dans la partie expérimentale.

La protection préalable du carboxy au niveau de R'a dans les réactifs de formules III, V, VI et VIII est notamment effectuée sous forme de dérivés de type esters, notamment d'alkyle, allyle, benzyle, benzhydryle ou p-nitro benzyle.

La déprotection peut être effectuée par différentes méthodes connues de l'homme du métier, par exemple par saponification, hydrolyse acide, hydrogénolyse ou clivage à l'aide de complexes solubles du Palladium 0.

La protection préalable de l'hydroxy du composé de formule (II) est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

Des illustrations des protections et déprotections ci-dessus sont fournies plus loin dans la partie expérimentale.

La base en présence de laquelle on fait réagir le composé de formule (II) et le diphosgène peut par exemple être une amine telle que la triéthylamine, mais d'autres bases connues de l'homme du métier pour des réactions de ce type peuvent être utilisées. On peut opérer au sein d'un solvant tel que le chlorure de méthylène.

La réaction de sulfatation est effectuée par action des complexes de SO₃ tels que SO₃-pyridine ou SO₃-diméthylformamide, en opérant dans la pyridine ou dans le diméthylformamide, le sel formé, par exemple le sel de pyridine, pouvant ensuite être échangé par exemple par un sel d'une autre amine, d'un ammonium quaternaire ou d'un métal alcalin. Une illustration est fournie dans la partie expérimentale.

La salification par les acides est le cas échéant réalisée par addition d'un acide en phase soluble au composé. La salification par les bases de la fonction sulfooxy peut être réalisée à partir du sel d'amine et notamment de pyridine obtenu lors de l'action du complexe SO₃-amine et on obtient les autres sels à partir de ce sel d'amine. On peut notamment opérer par échange d'ions sur résine.

La séparation des énantiomères et diastéréoisomères peut être réalisée selon les techniques connues de l'homme du métier, notamment la chromatographie, sur phase chirale ou non.

Des exemples de conditions utilisables sont aussi décrits dans la demande WO 04/052891 ou encore dans la demande WO 02/100860.

La base en présence de laquelle on fait réagir le composé de formule (II) préalablement à l'action du réactif de formule (V) peut par exemple être un hydrure alcalin tel que l'hydrure de sodium, mais d'autres bases connues de l'homme du métier pour des réactions de ce type peuvent être utilisées. La réaction peut être effectuée dans le tétrahydrofuranne.

La base en présence de laquelle on fait réagir le composé de formule (II) avec le réactif de formule (VI) dans lequel B représente un halogène peut par exemple être une amine telle que la diisopropyléthylamine ou la triéthylamine. On peut opérer au sein du diméthylformamide ou du dichlorométhane.

Les conditions dans lesquelles l'on fait réagir le composé de formule (II) avec le réactif de formule (VI) dans lequel B représente un OH, sont les conditions classiques des couplages peptidiques connues de l'homme du métier. De telles conditions sont illustrées plus loin dans la partie expérimentale.

La base en présence de laquelle on fait réagir le composé de formule (II) et le réactif de formule (VII) peut par exemple être une amine telle que la triéthylamine, mais d'autres bases connues de l'homme du métier pour des réactions de ce type peuvent être utilisées. On opère en présence de chlorure de mercure et au sein d'un solvant tel que le chlorure de méthylène.

La réaction du composé de formule (II) avec le réactif de formule (VIII) peut être effectuée au sein de l'acétonitrile ou d'un mélange avec le tétrahydrofuranne.

La réaction du composé de formule (II') avec le réactif de formule (IX) est effectuée en présence de triméthylphosphine et on opère par exemple au sein du tétrahydrofuranne ou d'un mélange tétrahydrofuranne/toluène.

L'invention a encore pour objet les composés intermédiaires de formules (IV), (IVa), (IVb), (IVc), (IVd) et (X) telles que définies plus haut.

Les composés de formule (II) et (II') peuvent être obtenus par des procédés décrits dans les demandes WO 02/100860_ou 04/052891.

Comme indiqué plus haut, les composés de formule générale (I) possèdent une excellente activité antibiotique sur *Pseudomonas aeruginosa* ainsi que sur des modèles d'infection animale par des souches résistantes aux agents antibactériens communément utilisés. Cette activité antibiotique remarquable et inattendue n'avait pas été observée pour les composés décrits dans la demande WO 04/052891 et notamment pour les composés structurellement proches. Ceci est illustré plus loin.

Ces propriétés rendent aptes lesdits composés, sous forme libre et de zwitterions ou de sels d'acides et de bases pharmaceutiquement acceptables, à être utilisés comme médicaments dans le traitement des infections sévères à *Pseudomonas,* notamment les infections nosocomiales et, d'une manière générale, les infections majeures chez les sujets à risques. Il peut en particulier s'agir d'infections des voies respiratoires, par exemple la pneumonie aiguë ou les infections chroniques des voies inférieures, les infections du sang, par exemple les septicémies, les infections aiguës ou chroniques des voies urinaires, celles du système auditif, par exemple l'otite externe maligne, ou l'otite chronique suppurante, celles de la peau et des tissus mous, par exemple les dermatites, les plaies infectées, la folliculite, la pyodermite, les formes rebelles d'acnée, les infections des yeux, par exemple l'ulcère de la cornée, celles du système nerveux, notamment les méningites et les abcès du cerveau, les infections cardiaques telles que l'endocardite, les infections des os et des articulations telles que la pyoarthrose sténoarticulaire, l'ostéomyélite vertébrale, la symphysite pubienne, les infections du tube gastro-intestinal, telles que l'entérocolite nécrosante et les infections péri-rectales.

La présente invention a donc également pour objet, à titre de médicaments et notamment de médicaments antibiotiques, les composés de formule (I) telle que définie ci-dessus, sous forme libre et sous forme de zwitterions et de sels avec les bases et les acides minéraux ou organiques pharmaceutiquement acceptables.

Parmi les composés de formule (I), l'invention a notamment pour objet, à titre de médicaments, les composés dans lesquels R₂ et R₃ forment ensemble un hétérocycle pyrazolyle substitué.

Parmi les composés de formule (I), l'invention a plus particulièrement pour objet, à titre de médicaments, ceux dans lesquels R₁ représente un radical -(CH₂)ₘ-NH₂, ainsi que ceux dans lesquels R₁ représente un radical -(CH₂)ₘ-NH-C(NH)NH₂, m dans l'un et l'autre cas étant égal à 1.

Parmi les composés de formule (I), l'invention a aussi notamment pour objet, à titre de médicaments, ceux dans lesquels R₄ représente une une chaîne de formule -(A)ₙ-(NH)ₒ-(CH₂)ₚ-(CHR')_{q} R" telle que définie précédemment et tout particulièrement ceux dans lesquels R₄ représente une chaîne de formule -C(O)-NH-(CH₂)ₚ-(CHR')_{q} R" dans laquelle R', R", p et q sont tels que définis plus haut.

Parmi les composés de formule (I), l'invention a encore notamment pour objet, à titre de médicaments, ceux dans lesquels R₄ représente un atome d'hydrogène ou un radical (C₁-C₆)alc et R₁ représente un radical -(CH₂)ₘ-NH-C(NH)NH₂ ou -(CH₂)ₘ-NH-CH=NH, dans lequel m est égal à 1.

Parmi les composés de formule (I), l'invention a tout particulièrement pour objet, à titre de médicaments, les composés décrits plus loin dans la partie expérimentale et notamment ceux dont les noms suivent:
- la trans 8-(aminométhyl)-2-(2-amino-éthyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-2-(4-pipérazine-1-carbonyl)-4,8-dihydro-5-(sulfooxy)-4,7-méthano-7H-pyrazolo[3,4-e] [1,3] diazépin-6(5H)-one,
- la trans 8-(aminométhyl)-4,8-dihydro-2-(2-diméthylamino-éthyl-carbamoyl)-5-(sulfooxy)-4,7-méthano-7H-pyrazolo[3,4-e] [1,3] diazépin-6(5H)-one,
- la trans 8-(aminométhyl)-2-(3-amino-propyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-2-(carbamoylméthyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-1-(carbanimidoyl)-4,8-dihydro-5-(sulfooxy)-4,7-méthano-7H-pyrazolo[3,4-e] [1,3] diazépin-6(5H)-one,
- la trans 8-(aminométhyl)-2-(carbanimidoyl)-4,8-dihydro-5-(sulfooxy)-4,7-méthano-7H-pyrazolo[3,4-e] [1,3] diazépin-6(5H)-one,
- la trans 8-(amino-méthyl)-4,8-dihydro-2-(pipéridine-4-carbonyl)-5-(sulfooxy)-4,7-méthano-7H-pyrazolo[3,4-e] [1,3] diazépin-6(5H)-one,
- la trans 8-(amino-méthyl)-2-(3-amino-3-carboxy-propyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-1-méthyl-5-(sulfooxy)-4,7-méthano-7H-pyrazolo[3,4-e] [1,3] diazépin-6(5H)-one,
- la trans 8-(guanidino-méthyl)-2-carbamoyl-4,8-dihydro-(sulfooxy)-4,7-méthano-7H-pyrazolo[3,4-e] [1,3] diazépin-6(5H)-one,
- la trans 8-(amino-méthyl)-4,8-dihydro-1-méthyl-5-(carboxy-difluoro-méthoxy)-4,7-méthano-7H-pyrazolo[3,4-e] [1,3] diazépin-6(5H)-one,
- la trans 8-(amino-méthyl)-2-(amino-carbamoyle)-4,8-dihydro-5-(sulfooxy)-4,7-méthano-7H-pyrazolo[3,4-e] [1,3] diazépin-6(5H)-one,
sous forme libre, de zwittterions et de sels avec les bases et les acides minéraux ou organiques pharmarceutiquement acceptables, et sous leurs formes isomères ou diastéroisomères possibles, ou de mélanges.

L'invention a aussi pour objet les compositions pharmaceutiques renfermant comme principe actif, au moins un des composés selon l'invention tels que définis ci-dessus. Ces compositions peuvent être administrées par voie buccale, rectale, parentérale, notamment intramusculaire, ou par voie locale en application topique sur la peau et les muqueuses.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'un lyophilisat destiné à être dissout extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1, 4 ou 5 ou encore comprise entre 0,25 g et 10 g par jour par voie intramusculaire ou intraveineuse.

Les produits de formule (I) peuvent également être utilisés comme désinfectants des instruments chirurgicaux.

Les exemples suivants illustrent l'invention.

### Exemple 1 : Sel de sodium et de trifluoroacétate de la trans 8-(aminométhyl)-2-(2-amino-éthylcarbamoyle)-4,8-dihydro-5-(sulfooxy)-4,7-méthano-7H-pyrazolo[3,4-e][1,3] diazépin-6(5H)-one

### Stade A

### trans 4,8-dihydro-8-(hydroxyméthyl)- 5-(phénylméthoxy)-4,7-méthano-7H-pyrazolo[3,4-e][1,3]diazépin-6(5H)-one

L'ester trans-4,5,6,8-tétrahydro-6-oxo-5-(phénylméthoxy)-4,7-méthano-7H-pyrazolo[3,4-e][1,3]diazépine-8-carboxylate de méthyle décrit dans la demande WO2004/052891 (5g, 15.2mmol) est mis en solution dans un mélange méthanol/tétrahydrofurane anhydres 1/1 (100mL), sous azote. Du NaBH₄ (2.3g, 60.9mmol) est alors ajouté par portions. Après une nuit d'agitation à température ambiante, le mélange réactionnel est traité avec une solution aqueuse 10% de NaH₂PO₄ (100mL). Après évaporation à sec, le mélange réactionnel est repris dans l'eau. Le précipité formé est agité une nuit dans la glace, puis filtré et séché au moins 24h sous vide en présence de P₂O₅, pour donner le composé attendu (3.30g, 1 1.0mmol, 72%) sous forme de poudre blanche.
MS (ES(+)) : m/z [M+H]⁺ = 301
¹H RMN (400MHz, DMSO-d₆) : δ(ppm) = 3.18-3.50 (ABX, 2H, N-CH₂-CH-N), 3.65-3.76 (ABX, 2H, N-CH-CH₂-OH), 4.34 (t, 1H, N-CH-CH₂-OH), 4.46 (d, 1H, N-CH₂-CH-N), 4.88 (s, 2H, CH₂-Ph), 7.29-7.43 (m, 5H, Ph), 7.66 (s, 1H, H pyrazole), 12.72 (broad, 1H, OH).

### Stade B

### trans [[4,5,6,8-tetrahydro-6-oxo-5-(phénylméthoxy)-4,7-méthano-7H-pyrazolo[3,4-e][1,3] diazépin-8-yl]méthyl]-carbamate de 1,1-diméthyléthyle

L'alcool obtenu au stade précédent (1.73g, 5.76mmol) est mis en solution dans la pyridine anhydre (35mL) sous azote, à 0°C. Puis du chlorure de méthanesulfonyle (1.78mL, 23mmol) est ajouté goutte à goutte. Après 2h30 d'agitation à température ambiante, le mélange réactionnel est traité avec une solution aqueuse saturée de chlorure d'ammonium (100mL), puis extrait à l'acétate d'éthyle. Les phases organiques combinées sont ensuite lavées 5 fois avec une solution aqueuse saturée de chlorure d'ammonium, séchées sur sulfate de sodium, filtrées puis concentrées sous vide pour donner le dérivé dimésylé attendu sous forme d'huile jaune.

L'intermédiaire dimésylé est mis en solution dans du diméthylformamide anhydre (45mL), sous azote, en présence d'azoture de sodium (1.12g, 17.3mmol). Le mélange réactionnel est chauffé à 70°C pendant 24h. Si nécessaire 1 éq. d'azoture est ajouté pour que la conversion soit complète. Lorsque la réaction est complète, le mélange est traité avec une solution aqueuse 10% de NaH₂PO₄ (100mL) puis extrait au dichlorométhane. Les phases organiques combinées sont séchées sur sulfate de sodium, filtrées puis concentrées sous vide pour donner l'azoture attendu sous forme d'huile jaune.

L'intermédiaire est mis en réaction, sous azote, dans l'éthanol absolu (17.5mL). Puis sont ajoutés successivement du di-tert-butyl dicarbonate (1.38g, 6.34mmol), du triéthylsilane (1.38mL, 8.64mmol) et de l'hydroxyde de palladium sur charbon 10% Degussa (52mg). Après une nuit à température ambiante, le mélange réactionnel est filtré puis concentré pour donner une huile jaune brute. Ce brut est purifié par chromatographie sur colonne de silice (éluant gradient CH₂Cl₂/MeOH 100/0 à 95/5 par 1%) pour conduire au composé attendu (1.36g, 3.40mmol, 34%) sous forme de solide blanc.
MS (ES(+)) : m/z [M+H]⁺= 401
¹H RMN (400MHz, MeOH-d₄) : δ(ppm) = 1.51 (s, 9H, C(CH₃)₃), 3.21-3.59 (m, 4H, N-CH₂-CH-N et N-CH-CH₂-NHBoc), 4.36 (m, 1H, N-CH-CH₂-OH), 4.46 (m, 1H, N-CH₂-CH-N), 4.99 (AB, 2H, CH₂-Ph), 7.41-7.52 (m, 5H, Ph), 7.63 (s, 1H, H pyrazole).

### Stade C

### trans [[2-(2-tert-butoxycarbonylamino-éthylcarbamoyle)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

Sous azote, le dérivé obtenu au stade précédent (170mg, 0.426mmol) est mis en solution dans le dichlorométhane (22mL). A 0°C, la triéthylamine (119µL, 0.851mmol) est ajoutée, suivie du diphosgène (77µL, 0.638mmol) ajouté par goutte à goutte rapide. Après 2h30 d'agitation à 0°C, la N-boc-éthylènediamine (236µL, 1.49mmol) est ajoutée rapidement et le milieu est agité vigoureusement à température ambiante pendant 1h.

Le milieu est transvasé dans une ampoule à décanter, rincé par du dichlorométhane (5mL), puis lavé par une solution aqueuse de phosphate de sodium à 10% (15mL). La phase aqueuse est extraite par le dichlorométhane (15mL). Les phases organiques sont rassemblées, lavées par une solution saturée de NaCl, séchées sur MgSO₄, concentrées sous vide pour donner, après chromatographie sur colonne de silice (éluant gradient CH₂Cl₂/acétate d'éthyle 90/10 à 80/20), le dérivé attendu (86mg, 0.147mmol, 35%).
MS (ES(+)) : m/z [M+H]⁺= 586
¹H NMR (400 MHz, CDCl₃) : δ (ppm) = 1.43 (s, 9H, C(CH₃)₃), 1.47 (s, 9H, C(CH₃)₃), 3.09 (dd, 1H, N-CH₂-CH-N), 3.29-3.40, 3.49-3.59 (m, 6H, CH-CH₂-NHBoc, N-CH₂-CH₂-N, N-CH₂-CH₂-N), 3.79 (dd, 1H, N-CH₂-CH-N), 3.98 (d,1H, N-CH₂-CH-N), 4.59 (m, 1H, CH-CH₂-NHBoc), 4.92 (AB, 2H, CH₂-Ph), 5.10 (broad, 1H, NH), 6.95 (broad, 1H, NH), 7.40-7.43 (m, 5H, Ph), 8.04 (s, 1H, H pyrazole).

### Stade D

### Sel de pyridinium du trans [[2-(2-tert-butoxycarbonylamino-éthylcarbamoyle)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

Sous azote, le dérivé obtenu au stade précédent (134mg, 0.229mmol) est mis en solution dans le diméthylformamide (0.33mL) et le dichlorométhane (0.98mL). Le palladium 10% sur charbon à 50% en eau (73mg, 0.034mmol) est ajouté. Après trois purges vide/azote, le mélange réactionnel est placé sous atmosphère d'hydrogène jusqu'à disparition du produit de départ en HPLC. Le mélange est alors concentré sous vide puis co-évaporé trois fois avec du dichlorométhane anhydre, enfin séché sous cloche à vide en présence de P₂O₅ pendant 2h, pour donner l'intermédiaire débenzylé attendu.

Le dérivé débenzylé est repris dans de la pyridine anhydre (0.9mL) en présence du complexe pyridine / sulfure de trioxyde (73mg, 0.458mmol). Le mélange réactionnel est agité à température ambiante jusqu'à conversion complète en HPLC, puis concentré à sec après traitement par ajout d'eau. Le brut réactionnel est chromatographié sur colonne de silice (éluant gradient CH₂Cl₂/MeOH 100/0 à 85/15 par 5%) pour donner le sel attendu (79mg, 0.121mmol, 53%).
MS (ES(-)) : m/z [M-H]⁻ = 574
¹H NMR (400MHz, MeOH-d₄): δ (ppm) = 1.25 (s, 9H, C(CH₃)₃), 1.32 (s, 9H, C(CH₃)₃), 3.05-3.16, 3.22-3.32, 3.39-3.54 (m, 8H, N-CH₂-CH-N, CH-CH₂-NHBoc, N-CH₂-CH₂-N, N-CH₂-CH₂-N), 4.40 (m, 1H, CH-CH₂-NHBoc), 4.78 (d,1H, N-CH₂-CH-N), 7.39 (broad, 1H, NH), 7.84 (m, 2H, Py), 8.07 (s, 1H, H pyrazole), 8.20 (broad, 1H, NH), 8.48(m, 1H, Py), 8.66 (m, 2H, Py), 9.02 (broad, 1H, NH)

### Stade E

### Sel de sodium du trans [[2-(2-tert-butoxycarbonylamino-éthylcarbamoyle)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

Une suspension de 10g de résine DOWEX 50WX8 dans une solution de soude 2N (50mL) est agitée pendant 1h, puis versée sur une colonne à chromatographie. La colonne est conditionnée à l'eau déminéralisée jusqu'à pH neutre, puis avec un mélange eau/THF 90/10. Le sel obtenu au stade précédent (79mg, 0.121mmol) est dissout dans un minimum de méthanol, déposé sur la colonne, puis élué avec un mélange eau/THF 90/10. Les fractions contenant le substrat sont réunies et congelées. La solution congelée est lyophilisée pour conduire au sel de sodium attendu (65mg, 0.109mmol, 90%) sous forme d'un solide beige.
MS (ES(-)) : m/z [M-H]⁻ = 574
¹H NMR (400MHz, DMSO-d₆): δ (ppm) = 1.36 (s, 9H, C(CH₃)₃), 1.41 (s, 9H, C(CH₃)₃), 3.05-3.13, 3.16-3.46 (m, 8H, N-CH₂-CH-N, CH-CH₂-NHBoc, N-CH₂-CH₂-N, N-CH₂-CH₂-N), 4.38 (m, 1H, CH-CH₂-NHBoc), 4.76 (d,1H, N-CH₂-CH-N), 6.93 (broad, 1H, NH), 7.13 (broad, 1H, NH), 8.17 (s, 1H, H pyrazole), 8.42 (broad, 1H, NH)

### Stade F

### Sel de sodium et de trifluoroacétate de la trans 8-(aminométhyl)-2-(2-amino-éthylcarbamoyle)-4,8-dihydro-5-(sulfooxy)-4,7-méthano-7H-pyrazolo[3,4-e][1,3] diazépin-6(5H)-one

Une solution d'acide trifluoroacétique (2.4mL) dans le dichlorométhane (2.4mL) est ajoutée goutte à goutte à une solution du sel de sodium obtenu au stade précédent (42mg, 0.092mmol) dans le dichlorométhane (1.2mL) sous azote et refroidie à 0°C. La réaction est maintenue sous agitation pendant 1h à température ambiante. Le mélange est évaporé à sec. Le résidu est repris dans l'eau (3mL) et la solution est lavée par l'éther diéthylique (3mL). La solution aqueuse est congelée puis lyophilisée pour donner le sel de sodium et de trifluoroacétate attendu (62mg, 0.099mmol, 94%) sous forme d'un solide beige.
MS (ES(-)) : m/z [M-H]⁻ = 374
¹H NMR (400 MHz, DMSO-d₆) : δ (ppm) = 2.98-3.70 (m, 8H, N-CH₂-CH-N, CH-CH₂-NH₃⁺, N-CH₂-CH₂-N, N-CH₂-CH₂-N), 4.72 (m, 1H, CH-CH₂-NH₃⁺), 4.88 (d, 1H, N-CH₂-CH-N), 7.76 (broad, 3H, NH₃⁺), 8.16 (broad, 3H, NH₃⁺), 8.32 (s, 1H, H pyrazole), 8.59 (broad, 1H, NH)

### Exemple 2 : Sel de sodium et de trifluoroacétate de la trans 8-(aminométhyl)-2-(4-pipérazine-1-carbonyl)-4,8-dihydro-5-(sulfooxy)-4,7-méthano-7H-pyrazolo[3,4-e][1,3]diazépin-6(5H)-one

### Stade A

### trans [[2-(4-tert-butoxycarbonyl-pipérazine-1-carbonyl)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

En procédant comme indiqué au stade C de l'exemple 1, le dérivé obtenu au stade précédent (150mg, 0.376mmol), le dichlorométhane (20mL), la triéthylamine (105µL, 0.751mmol), le diphosgène (68µL, 0.563mmol) et la N-boc-pipérazine (210mg, 1.13mmol) conduisent, après chromatographie sur colonne de silice (éluant gradient CH₂Cl₂/acétate d'éthyle 80/20 à 70/30), au dérivé attendu (145mg, 0.237mmol, 63%) sous forme d'un solide beige.
MS (ES (+)) : m/z [M+H]⁺= 612
¹H NMR (400 MHz, CDCl₃) : δ(ppm) = 1.45 (s, 9H, C(CH₃)₃), 1.48 (s, 9H, C(CH₃)₃), 3.07 (dd, 1H, N-CH₂-CH-N), 3.33 (m, 2H, CH-CH₂-NHBoc), 3.51 (m, 4H, N-CH₂-CH₂-N), 3.77 (m, 5H, N-CH₂-CH₂-N, N-CH₂-CH-N), 4.00 (d,1H, N-CH₂-CH-N), 4.60 (m, 1H, CH-CH₂-NHBoc), 4.92 (AB, 1H, CH₂-Ph), 5.13 (broad, 1H, NH), 7.38-7.43 (m, 5H, Ph), 7.98 (s, 1H, H pyrazole).

### Stade B

### Sel de pyridinium du trans [[2-(4-tert-butoxycarbonyl-pipérazine-1-carbonyl)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade D de l'exemple 1, le dérivé obtenu au stade précédent (145mg, 0.237mmol), le diméthylformamide (0.34mL), le dichlorométhane (1.0mL) et le palladium 10% sur charbon à 50% en eau (76mg, 0.036mmol) conduisent à l'intermédiaire débenzylé attendu.

L'intermédiaire débenzylé, la pyridine (1.0mL) et le complexe pyridine / sulfure de trioxyde (112mg, 0.702mmol) conduisent, après chromatographie sur colonne de silice (éluant gradient CH₂Cl₂/MeOH 100/0 à 80/20 par 5%) au dérivé attendu (40mg, 0.059mmol, 21%) sous forme d'un solide beige.
MS (ES(-)) : m/z [M-H]⁻ = 600
¹H NMR (400MHz, CDCl₃): δ (ppm) = 1.42 (s, 9H, C(CH₃)₃), 1.47 (s, 9H, C(CH₃)₃), 3.02-3.28, 3.40-3.90 (m, 12H, N-CH₂-CH-N, CH-CH₂-NHBoc, N-CH₂-CH₂-N, N-CH₂-CH₂-N), 4.58 (m, 1H, CH-CH₂-NHBoc), 4.97 (d,1H, N-CH₂-CH-N), 7.28 (s, 1H, H pyrazole), 8.12 (broad, 1H, NH)

### Stade C

### Sel de sodium du trans [[2-(4-tert-butoxycarbonyl-pipérazine-1-carbonyl)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade E de l'exemple 1, le sel obtenu au stade précédent (40mg, 0.059mmol), la résine DOWEX 50WX8 (5g) et la soude 2N (25mL) conduisent au sel de sodium attendu (32mg, 0.051mmol, 87%) sous forme d'un solide beige.
MS (ES(-)) : m/z [M-H]⁻ = 600
¹H NMR (400MHz, DMSO-d₆): δ (ppm) = 1.40 (s, 9H, C(CH₃)₃), 1.41 (s, 9H, C(CH₃)₃), 3.26-3.65 (m, 12H, N-CH₂-CH-N, CH-CH₂-NHBoc, N-CH₂-CH₂-N, N-CH₂-CH₂-N), 4.42 (m, 1H, CH-CH₂-NHBoc), 4.78 (d, 1H, N-CH₂-CH-N), 7.06 (broad, 1H, NH), 8.13 (s, 1H, H pyrazole)

### Stade D

### Sel de sodium et de trifluoroacétate de la trans 8-(aminométhyl)-2-(pipérazine-1-carbonyl)-4,8-dihydro-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (126mg, 0.203mmol), le dichlorométhane (3.6mL), l'acide trifluoroacétique (7.2mL) dans le dichlorométhane (7.2mL) conduisent au sel de sodium et de trifluoroacétate attendu (124mg, 0.191mmol, 95%) sous forme d'un solide beige.
¹H NMR (400 MHz, DMSO-d₆) : δ (ppm) = 3.18-3.27, 3.37-3.43, 3.84-3.93 (m, 12H, N-CH₂-CH-NH₃⁺, CH-CH₂-N, N-CH₂-CH₂-N, N-CH₂-CH₂-N), 4.69 (m, 1H, CH-CH₂-NH₃⁺), 4.88 (d, 1H, N-CH₂-CH-N), 8.13 (broad, 3H, NH₃⁺), 8.24 (s, 1H, H pyrazole), 9.00 (broad, 2H, NH₂⁺)

### Exemple 3 : Sel de sodium et de trifluoroacétate de la trans 8-(aminométhyl)-4,8-dihydro-2-(2-diméthylaminoéthyl carbamoyle)-5-(sulfooxy)-4,7-méthano-7H-pyrazolo[3,4-e][1,3] diazépin-6(5H)-one

### Stade A

### trans [[2-(2-diméthylamino-éthylcarbamoyle)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

En procédant comme indiqué au stade C de l'exemple 1, le dérivé obtenu au stade B de l'exemple 1 (228mg, 0.571mmol), le dichlorométhane (30mL), la triéthylamine (159µL, 1.14mmol), le diphosgène (103µL, 0.856mmol) et la N,N-diméthyl-ethylènediamine (317µL, 2.85mmol) conduisent, après chromatographie sur colonne de silice (éluant gradient CH₂Cl₂/MeOH 100/0 à 95/5 par 1%), au dérivé attendu (193mg, 0.375mmol, 66%) sous forme d'un solide jaune.
MS (ES(+)) : m/z [M+H] ⁺= 514
¹H NMR (400 MHz, CDCl₃) : δ (ppm) = 1.46 (s, 9H, C(CH₃)₃), 2.30 (s, 3H, CH₃), 2.31 (s, 3H, CH₃), 2.53 (m, 2H, N-CH₂-CH₂-NMe₂), 3.08 (dd, 1H, N-CH₂-CH-N), 3.38 (m, 2H, CH-CH₂-NHBoc), 3.48 (m, 2H, N-CH₂-CH₂-NMe₂), 3.80 (dd, 1H, N-CH₂-CH-N), 3.98 (d,1H, N-CH₂-CH-N), 4.60 (m, 1H, CH-CH₂-NHBoc), 4.92 (AB, 2H, CH₂-Ph), 5.16 (broad, 1H, NH), 7.39-7.43 (m, 5H, Ph), 8.04 (s, 1H, H pyrazole).

### Stade B

Sel de pyridinium du *trans* [[2-(2-diméthylamino-éthylcarbamoyle)-4,5,6,8-tétrahydro-6-oxo-5-(sulfooxy)-4,7-méthano-7*H-*pyrazolo[3,4-e][1,3] diazépin-8-yl]méthyl]-carbamate de 1,1-diméthyléthyle.

En procédant comme indiqué au stade D de l'exemple 1, le dérivé obtenu au stade précédent (173mg, 0.337mmol), le diméthylformamide (0.48mL), le dichlorométhane (1.44mL) et le palladium 10% sur charbon à 50% en eau (108mg, 0.051mmol) conduisent à l'intermédiaire débenzylé attendu.

L'intermédiaire débenzylé, la pyridine (1.1mL) et le complexe pyridine / sulfure de trioxyde (107mg, 0.673mmol) conduisent, après chromatographie sur colonne de silice (éluant gradient CH₂Cl₂/MeOH 100/0 à 85/15 par 5%) au dérivé attendu (107mg, 0.184mmol, 55%) sous forme d'un solide beige.
MS (ES(-)) : m/z [M-H]⁻ = 574
¹H NMR (400MHz, MeOH-d₄): δ (ppm) = 1.52 (s, 9H, C(CH₃)₃), 3.04 (s, 6H, 2 x CH₃), 3.48 (m, 4H, CH-CH₂-NHBoc, N-CH₂-CH₂-NMe₂), 3.61 (d, 1H, N-CH₂-CH-N), 3.72 (dd, 1H, N-CH₂-CH-N), 4.82 (m, 2H, N-CH₂-CH₂-NMe₂), 4.65 (m, 1H, CH-CH₂-NHBoc), 4.95 (d,1H, N-CH₂-CH-N), 8.28 (s, 1H, H pyrazole)

### Stade C

### Sel de sodium du trans [[2-(2-diméthylamino-éthylcarbamoyle)- 4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade E de l'exemple 1, le sel obtenu au stade précédent (107mg, 0.184mmol), la résine DOWEX 50WX8 (13g) et la soude 2N (65mL) conduisent au sel de sodium attendu (87mg, 0.166mmol, 91%) sous forme d'un solide beige.
MS (ES (+)) : m/z [M+H]⁺ = 504
¹H NMR (400MHz, DMSO-d₆): δ (ppm) = 1.41 (s, 9H, C(CH₃)₃), 3.24-3.43 (m, 14H, 2 x CH₃, CH-CH₂-NHBoc, N-CH₂-CH₂-NMe₂, N-CH₂-CH-N, N-CH₂-CH₂-NMe₂), 4.40 (m, 1H, CH-CH₂-NHBoc), 4.78 (d,1H, N-CH₂-CH-N), 7.15 (broad, 1H, NH), 8.20 (s, 1H, H pyrazole)

### Stade D

### Sel de sodium et de trifluoroacétate de la trans 8-(aminométhyl)-4,8-dihydro-2-(2-diméthylamino-éthylcarbamoyle)-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (87mg, 0.166mmol), le dichlorométhane (2.5mL), l'acide trifluoroacétique (5.0mL) dans le dichlorométhane (5.0mL) conduisent au sel de sodium et de trifluoroacétate attendu (76mg, 0.141mmol, 85%) sous forme d'un solide beige.
MS (ES(-)) : m/z [M-H]⁻ = 402
¹H NMR (400 MHz, DMSO-d₆) : δ (ppm) = 2.17 (s, 6H, 2 x CH3), 3.12-3.72 (m, 8H, N-CH₂-CH-N, CH-CH₂-NH₃⁺, N-CH₂-CH₂-NMe₂, N-CH₂-CH₂-NMe₂), 4.73 (dd, 1H, CH-CH₂-NH₃⁺), 4.88 (d, 1H, N-CH₂-CH-N), 8.17 (broad, 3H, NH₃⁺), 8.32 (s, 1H, H pyrazole), 8.63 (broad, 1H, NH)

### Exemple 4 : Sel de sodium et de trifluoroacétate de la trans 8-(aminométhyl)-4,8-dihydro-2-(pyrrolidine-1-carbonyl)-5-(sulfooxy)-4,7-méthano-7H-pyrazolo[3,4-e][1,3] diazépin-6(5H)-one

### Stade A

### Trans [[4,5,6,8-tétrahydro-6-oxo-5-(phénylméthoxy)-2-(pyrrolidine-1-carbonyl)-4,7-méthano-7H-pyrazolo[3,4-e][1,3] diazépin-8-yl]méthyl]-carbamate de 1,1-diméthyléthyle

En procédant comme indiqué au stade C de l'exemple 1, le dérivé obtenu au stade B de l'exemple 1 (150mg, 0.376mmol), le dichlorométhane (20mL), la triéthylamine (105µL, 0.751mmol), le diphosgène (68µL, 0.563mmol) et la pyrrolidine (157µL, 1.88mmol) conduisent, après chromatographie sur colonne de silice (éluant CH₂Cl₂/acétate d'éthyle 85/15), au dérivé attendu (143mg, 0.288mmol, 76%) sous forme d'un solide beige.
MS (ES(+)) : m/z [M+H]⁺= 497
¹H NMR (400 MHz, CDCl₃) : δ (ppm) = 1.47 (s, 9H, C(CH₃)₃), 1.89 (m, 4H, N-CH₂-CH₂pyrrolidine), 3.08 (dd, 1H, N-CH₂-CH-N), 3.36 (dd, 1H, N-CH₂-CH-N), 3.45 (m, 1H, CH-CH₂-NHBoc), 3.65 (m, 2H, N-CH₂-CH₂-Npyrrolidine), 3.79 (m, 1H, CH-CH₂-NHBoc), 3.92 (m, 2H, N-CH₂-CH₂-Npyrrolidine), 3.99 (d, 1H, N-CH₂-CH-N), 4.63 (m, 1H, CH-CH₂-NHBoc), 4.94 (AB, 2H, CH₂-Ph), 5.16 (broad, 1H, NH), 7.39-7.43 (m, 5H, Ph), 8.11 (s, 1H, H pyrazole).

### Stade B

### Sel de pyridinium du trans [[4,5,6,8-tétrahydro-6-oxo-2-(pyrrolidine-1-carbonyl)-5-(sulfooxy)-4,7-méthano-7H-pyrazolo[3,4-e][1,3] diazépin-8-yl]méthyl]-carbamate de 1,1-diméthyléthyle

Sel de pyridinium du trans [[4,8-dihydro-6-oxo-2-En procédant comme indiqué au stade D de l'exemple 1, le dérivé obtenu au stade précédent (143mg, 0.288mmol), le diméthylformamide (0.41mL), le dichlorométhane (1.23mL) et le palladium 10% sur charbon à 50% en eau (92mg, 0.043mmol) conduisent à l'intermédiaire débenzylé attendu.

L'intermédiaire débenzylé, la pyridine (0.93mL) et le complexe pyridine / sulfure de trioxyde (88mg, 0.553mmol) conduisent, après chromatographie sur colonne de silice (éluant gradient CH₂Cl₂/MeOH 100/0 à 85/15 par 5%) au dérivé attendu (76mg, 0.134mmol, 49%) sous forme d'un solide beige.
MS (ES(-)) : m/z [M-H]⁻ = 485
¹H NMR (400MHz, MeOH-d₄): δ (ppm) = 1.51 (s, 9H, C(CH₃)₃), 2.00 (m, 4H, N-CH₂-CH₂pyrrolidine), 3.49 (dd, 1H, N-CH₂-CH-N), 3.57 (dd, 1H, N-CH₂-CH-N), 3.65 (m, 4H, N-CH₂-CH₂-Npyrrolidine), 3.96 (m, 2H, CH-CH₂-NHBoc), 4.67 (m, 1H, CH-CH₂-NHBoc), 4.98 (d,1H, N-CH₂-CH-N), 8.30 (s, 1H, H pyrazole)

### Stade C

Sel de sodium du *trans* [[4,5,6,8-tétrahydro-6-oxo-2-(pyrrolidine-1-carbonyl)-5-(sulfooxy)-4,7-méthano-7*H-*pyrazolo[3,4-e][1,3] diazépin-8-yl]méthyl]-carbamate de 1,1-diméthyléthyle En procédant comme indiqué au stade E de l'exemple 1, le dérivé obtenu au stade précédent (76mg, 0.134mmol), la résine DOWEX 50WX8 (9g) et la soude 2N (45mL) conduisent au sel de sodium attendu (64mg, 0.126mmol, 93%) sous forme d'un solide beige.
MS (ES(+)) : m/z [M+H]⁺ = 486
¹H NMR (400MHz, DMSO-d₆): δ (ppm) = 1.39 (s, 9H, C(CH₃)₃), 1.84 (m, 4H, N-CH₂-CH₂pyrrolidine), 3.25-3.40 (m, 4H, N-CH₂-CH-N, N-CH₂-CH₂-Npyrrolidine), 3.54 (m, 2H, N-CH₂-CH₂-Npyrrolidine), 3.79 (m, 2H, CH-CH₂-NHBoc), 4.41 (m, 1H, CH-CH₂-NHBoc), 4.78 (d, 1H, N-CH₂-CH-N), 7.05 (broad, 1H, NH), 8.16 (s, 1H, H pyrazole)

### Stade D

### Sel de sodium et de trifluoroacétate de la trans-8-(aminométhyl)-4,8-dihydro-2-(pyrrolidine-1-carbonyl)-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (64mg, 0.126mmol), le dichlorométhane (1.8mL), l'acide trifluoroacétique (3.7mL) dans le dichlorométhane (3.7mL) conduisent au sel de sodium et de trifluoroacétate attendu (57mg, 0.109mmol, 86%) sous forme d'un solide beige.
MS (ES(-)) : m/z [M-H]⁻ = 385
¹H NMR (400 MHz, DMSO-d₆) : δ (ppm) = 1.85 (m, 4H, N-CH₂-CH₂pyrrolidine), 3.42-3.74 (m, 8H, N-CH₂-CH-N, N-CH₂-CH₂-Npyrrolidine, CH-CH₂-NH₃⁺), 4.68 (m, 1H, CH-CH₂-NH₃⁺), 4.86 (d, 1H, N-CH₂-CH-N), 8.09 (broad, 3H, NH₃⁺), 8.26 (s, 1H, H pyrazole)

### Exemple 5 : Sel de sodium et de trifluoroacétate de la trans 8-(aminométhyl)-2-[(3-amino-propyl)carbamoyle]-4,8-dihydro-5-(sulfooxy)-4,7-méthano-7H-pyrazolo[3,4-e][1,3] diazépin-6(5H)-one

### Stade A

*Trans* 2-[(3*-tert-*butoxycarbonylamino-propyl)carbamoyle]-4,5,6,8-tétrahydro-5-(phénylméthoxy)-4,7-méthano-7*H*-pyrazolo[3,4-e][1,3] diazépin-8-yl]méthyl]-carbamate de 1,1-diméthyléthyle.

En procédant comme indiqué au stade C de l'exemple 1, le dérivé obtenu au stade B de l'exemple 1 (250mg, 0.626mmol), le dichlorométhane (32.8mL), la triéthylamine (174µL), le diphosgène (113µL)et la N-Boc-1,3-propanediamine (382µL) conduisent, après un temps de réaction de 3h30 (∼40% conversion) et chromatographie sur colonne de silice (éluant gradient CH₂Cl₂/AcOEt 100/0 à 70/30), au dérivé attendu (132mg, 0.22mmol, 35%).
MS (ES(+)) : m/z [M+H]⁺ = 600
¹H RMN (400MHz, CDCl₃) : δ(ppm) = 1.48 (s, 9H, ^{t}Bu), 1.61 (s, 9H, ^{t}Bu), 1.79 (m, 2h, NHCH₂-CH₂-CH₂-NHBOC), 3.05-3.75 (m + 2 ABX, 8H, N-CH₂-CH-N, N-CH-CH₂-NHBOC, NHCH₂-CH₂-CH₂-NHBOC, NHCH₂-CH₂-CH₂-NHBOC), 4.00 (d, 1H, N-CH-CH₂-NHBOC), 4.62 (dd, 1H, N-CH₂-CH-N), 4.97 (AB, 2H, CH₂-Ph), 7.42-7.45 (m, 5H, Ph), 8.05 (s, 1H, H pyrazole).

### Stade B

### Sel de sodium du trans 2-[(3-tert-butoxycarbonylamino-propyl)carbamoyle]-4,5,6,8-tétrahydro-5-(sulfooxy)-4,7-méthano-7H-pyrazolo[3,4-e][1,3] diazépin-8-yl]méthyl]-carbamate de 1,1-diméthyléthyle

En procédant comme indiqué au stade D de l'exemple 1, le dérivé obtenu au stade précédent (132mg, 0.22mmol), le mélange diméthylformamide / dichlorométhane 1/3 (2.5mL) et le palladium 10% sur charbon à 50% en eau (53 mg) conduisent à l'intermédiaire débenzylé attendu.

L'intermédiaire débenzylé, la pyridine (0.86mL) et le complexe pyridine / sulfure de trioxyde (70mg, 0.44mmol) conduisent, après chromatographie sur colonne de silice (éluant gradient CH₂Cl₂/MeOH 100/0 à 80/20) au dérivé attendu (90mg, 0.134mmol, 61%) sous forme d'un solide blanc.

En procédant comme indiqué au stade E de l'exemple 1, le sel obtenu au stade précédent (90mg, 0.134mmol), la résine DOWEX 50WX8 (11g) et la soude 2N (55mL) conduisent après élution à l'eau uniquement et lyophilisation au sel de sodium attendu (48mg, 0.078mmol, 58%) sous forme d'une poudre blanche.
MS (ES (-)) : m/z [M-H]⁻ = 588
¹H NMR (400MHz, MeOH-d₄): δ (ppm) = 1.49 (s, 9H, C(CH₃)₃), 1.52 (s, 9H, C(CH₃)₃), 1.81 (m, 2H, NCH₂-CH₂-CH₂-NHBoc), 3.18 (m, 2H, NCH₂-CH₂-CH₂-NHBoc), 3.36-3.45 (m, 6H, NCH₂-CH₂-CH₂-NHBoc ,N-CH₂-CH-N et CH-CH₂-NHBoc), 4.65 (dd, 1H, CH-CH₂-NHBoc), 4.99 (d, 1H, N-CH₂-CH-N), 8.29 (s, 1H, H pyrazole).

### Stade C

### Sel de sodium et de trifluoroacétate de la trans 8-(aminométhyl)-2[(3-amino-propyl)carbamoyle]-4,8-dihydro-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (48mg, 0.078mmol), le dichlorométhane (1.34mL), le mélange acide trifluoroacétique / dichlorométhane (5.36mL) conduisent au sel de sodium et de trifluoroacétate attendu (45mg, 0.070mmol, 90%) sous forme d'une poudre beige.
MS (ES (-)) : m/z [M-H]⁻ = 390
¹H NMR (400MHz, MeOH-d₄): δ (ppm) = 1.86 (m, 2H, NCH₂-CH₂-CH₂-NH₂), 2.91 (m, 2H, NCH₂-CH₂-CH₂-NH₂), 3.29-3.80 (m, 6H, NCH₂-CH₂-CH₂-NH₂ ,N-CH₂-CH-N et CH-CH₂-NH₂), 4.65 (dd, 1H, CH-CH₂-NH₂), 4.86 (d, 1H, N-CH₂-CH-N), 8.18 (s, 1H, H pyrazole).

### Exemple 6 : Sel de sodium et de trifluoroacétate de la trans 8-(aminométhyl)-2-[(3-amino-butyl)carbamoyle]-4,8-dihydro-5-(sulfooxy)-4,7-méthano-7H-pyrazolo[3,4-e][1,3] diazépin-6(5H)-one

### Stade A

### Trans 2-[(3-tert-butoxycarbonylamino-butyl)carbamoyle]-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

En procédant comme indiqué au stade C de l'exemple 1, le dérivé obtenu au stade B de l'exemple 1 (250mg, 0.626mmol), le dichlorométhane (32.8mL), la triéthylamine (174µL), le diphosgène (113µL)et la N-Boc-1,3-butanediamine (419µL) conduisent, après un temps de réaction de 3h30 (∼50% conversion) et chromatographie sur colonne de silice (éluant gradient CH₂Cl₂/AcOEt 100/0 à 70/30), au dérivé attendu (91mg, 0.148mmol, 24%).
MS (ES(+)) : m/z [M+H]⁺ = 614
¹H RMN (400MHz, CDCl₃) : δ(ppm) = 1.47 (s, 9H, ^{t}Bu), 1.48 (s, 9H, ^{t}Bu), 1.58-1.68 (m, 4H, NHCH₂-CH₂-CH₂-CH₂-NHBoc et NHCH₂-CH₂-CH₂-CH₂-NHBoc), 3.05-3.55 (m + 2 ABX, 8H, N-CH₂-CH-N, N-CH-CH₂-NHBoc, NHCH₂-CH₂-CH₂-CH₂-NHBoc, NHCH₂-CH₂-CH₂-CH₂-NHBoc), 4.00 (d, 1H, N-CH-CH₂-NHBoc), 4.60 (dd, 2H, N-CH₂-CH-N), 4.97 (AB, 2H, CH₂-Ph), 5.05 (sl, 1H, NH), 7.05 (sl, 1H, NH), 7.42-7.45 (m, 5H, Ph), 8.05 (s, 1H, H pyrazole).

### Stade B

### Sel de sodium du trans 2-[(3-tert-butoxycarbonylamino-butyl)carbamoyle]-4,5,6,8-tétrahydro-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, le dérivé obtenu au stade précédent (91mg, 0.15mmol), le mélange diméthylformamide / dichlorométhane 1/3 (1.7mL) et le palladium 10% sur charbon à 50% en eau (36 mg) conduisent à l'intermédiaire débenzylé attendu.

L'intermédiaire débenzylé, la pyridine (0.86mL) et le complexe pyridine / sulfure de trioxyde (47mg) conduisent, après chromatographie sur colonne de silice (éluant gradient CH₂Cl₂/MeOH 95/5 à 90/10) au dérivé attendu (38mg, 0.056mmol, 37%).

En procédant comme indiqué au stade E de l'exemple 1, le sel obtenu au stade précédent (38mg, 0.056mmol), la résine DOWEX 50WX8 (4.6g) et la soude 2N (23mL) conduisent après élution à l'eau uniquement et lyophilisation au sel de sodium attendu (43mg, 0.068mmol, 100%) sous forme d'une poudre blanche.
MS (ES (-)) : m/z [M-H]⁻ = 602
¹H RMN (400MHz, MeOD-d₄) : δ(ppm) = 1.50 (s, 9H, ^{t}Bu), 1.53 (s, 9H, ^{t}Bu), 1.59 (m, 2H, NHCH₂-CH₂-CH₂-CH₂-NHBoc), 1.68 (m, 2H, NHCH₂-CH₂-CH₂-CH₂-NHBoc), 3.11-3.82 (m + 2 ABX, 8H, N-CH₂-CH-N, N-CH-CH₂-NHBoc, NHCH₂-CH₂-CH₂-CH₂-NHBoc, NHCH₂-CH₂-CH₂-CH₂-NHBoc), 4.45 (sl, 1H, NH), 4.62 (dd, 1H, N-CH-CH₂-NHBoc), 5.00 (dd, 2H, N-CH₂-CH-N), 5.45 (sl, 1H, NH), 8.05 (s, 1H, H pyrazole), 8.38 (sl, 1H, NH).

### Stade C

### Sel de sodium et de trifluoroacétate de la trans 8-(aminométhyl)-2[(3-amino-butyl)carbamoyle]-4,8-dihydro-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent 43mg, 0.068mmol), le dichlorométhane (1.17mL), le mélange acide trifluoroacétique / dichlorométhane (4.68mL) conduisent au sel de sodium et de trifluoroacétate attendu (40mg, 0.064mmol, 93%) sous forme d'une poudre beige.
MS (ES (+)) : m/z [M+H]⁻ = 404
¹H RMN (400MHz, MeOD-d₄) : δ(ppm) = 1.20 (m, 2H, NHCH₂-CH₂-CH₂-CH₂-NH₂), 1.60 (m, 2H, NHCH₂-CH₂-CH₂-CH₂-NH₂), 2.70-3.72 (m + 2 ABX, 8H, N-CH₂-CH-N, N-CH-CH₂-NH₂, NHCH₂-CH₂-CH₂-CH₂-NH₂, NHCH₂-CH₂-CH₂-CH₂-NH₂), 4.80 (dd, 2H, N-CH₂-CH-N), 8.20 (s, 1H, H pyrazole).

### Exemple 7 : Sel de sodium et de trifluoroacétate de la trans 8-(aminométhyl)-2-(carbamoylméthyl-carbamoyl)-4,8-dihydro-5-(sulfooxy)-4,7-méthano-7H-pyrazolo[3,4-e] [1,3] diazépin-6(5H)-one

### Stade A

### trans [[2-(carbamoylméthyl-carbamoyl)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

En procédant comme indiqué au stade C de l'exemple 1, le dérivé obtenu au stade B de l'exemple 1 (200 mg, 0.501mmol), le dichlorométhane (26mL), la triéthylamine (0.489mL, 3.507mmol), le diphosgène (0.091mL, 0.751mmol) et le sel chlorhydrate de glicinamide (0.277mg, 2.50mmol) conduisent, après 2h de réaction et chromatographie sur colonne de silice de ce brut combiné avec un autre obtenu à partir de 50 mg de substrat de départ (0.125mmol) (éluant gradient CH₂Cl₂/AcOEt 100/0 à 00/100), au dérivé attendu (115mg, 0.23mmol, 36.8%).
MS (ES(+): m/z [M+H]⁺ = 500
¹H NMR (400 MHz, CDCl₃) : δ (ppm) = 1.48 (s, 9H, C(CH₃)₃), 3.10-3.20(AB, 1H, N-CH₂-CH-N), 3.36-3.39 (m, 2H, N-CH₂-CH-N et CH-CH₂-NHBoc), 3.8 (broad, 1H, CH-CH₂-NHBoc), 4.03 (d, 1H, N-CH₂-CH-N), 4.90(m, 2H , N-CH₂-CO-N), 4.60-4.70(broad, 1H,N-CH-CH₂-NHBoc), 4.88-5.05 (AB, 2H, N-O-CH₂-Ph), 5.20 (broad, 1H , NH), 5.65 (broad, 1H, CO-NH₂), 6.15(broad, 1H, CO-NH₂), 7.42- 7.45 (m, 5H, Ph), 7.65(broad, 1H, NH), 8.07 (s, 1H, H pyrazole).

### Stade B

### Sel de pyridinium du trans [[2-(carbamoylméthyl-carbamoyl)- 4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, le dérivé obtenu au stade précédent (110mg, 0.22mmol), le diméthylformamide (0.32mL), le dichlorométhane (0.96mL) et le palladium 10% sur charbon à 50% en eau (70.3mg, 0.033mmol) conduisent à l'intermédiaire débenzylé attendu. (MS (ES(+) : m/z [M+H]⁺ = 409)

L'intermédiaire débenzylé, la pyridine (0.69mL) et le complexe pyridine / sulfure de trioxyde (70mg, 0.440mmol) conduisent, après chromatographie sur colonne de silice (4g, éluant CH₂Cl₂/MeOH 100/0 à 80/20) au produit attendu mais avec une pureté encore insuffisante. celui-ci est remis en solution dans l'eau. La solution aqueuse est extraite par du dichlorométhane (2mL, 3 fois), puis la phase aqueuse est congelée et lyophilisée pour donner le composé attendu (65mg, 0.114mmol, 52%) sous forme d'un solide blanc.
MS ((ES(-)): m/z [M-H]⁻ = 488
¹H NMR (400 MHz, DMSO-*d*₆₊ 1 goutte D₂O) : δ (ppm) = 1.38 (s, 9H, C(CH₃)₃), 3.30-3.44(m, 4H, N-CH₂-CH-N et CH-CH₂-NHBoc), 3.67-4.00 (m, 2H , N-CH₂-CO-N), 4.40(m, 1H, N-CH₂-CH-N), 4.79(broad, 1H,N- CH-CH₂-NHBoc), 7.83- 7.84 (m, 2H, Pyridine), 8.20 (s, 1H, H pyrazole); 8.32-8.34 (m, 1H, Pyridine) ; 8.70-8.78 (m, 2H, pyridine).

### Stade C

### Sel de sodium du trans [[2-(carbamoylméthyl-carbamoyl)-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade E de l'exemple 1, le sel obtenu au stade précédent (55mg, 0.097mmol), la résine DOWEX 50WX8 (8 g) et la soude 2N (30mL) conduisent après dépôt du produit en solution dans l'eau et lyophilisation au sel de sodium attendu (38mg, 0.074mmol, 77%) sous forme de lyophilisat blanc
MS ((ES(-)): m/z [M-H]⁻ = 488)
¹H NMR (400 MHz, DMSO-d₆) : δ (ppm) = 1.40 (s, 9H, C(CH₃)₃); 3.32-3.40(m, 4H, N-CH₂-CH-N et CH-CH₂-NHBoc), 3.78 (m, 2H , N-CH₂-CO-N), 4.40-4.50(m, 1H, N-CH₂-CH-N), 4.75(m, 1H,N- CH-CH₂-NHBoc), 7.10-7.20(m, 2H , NH et NH), 8.19 (s, 1H, H pyrazole), 8.45 (m, 1H, NH).

### Stade D

### Sel de sodium et de trifluoroacétate de la trans-8-(aminométhyl)-2-(carbamoylméthyl-carbamoyl)-4,8-dihydro-5-(sulfooxy)-4,7-méthano-7H-pyrazolo[3,4-e][1,3]diazépin-6(5H)-one

En procédant comme indiqué au stade F de l'exemple 1, le sel de sodium obtenu au stade précédent (35mg, 0.068mmol), le dichlorométhane (3mL), l'acide trifluoroacétique (1mL) conduisent au sel de sodium et de trifluoroacétate attendu (34mg, 0.054mmol, 80%) sous forme de lyophilisat blanc.
MS (ES(+)) : m/z [M+H]⁺ = 390

### Exemple 8: Sel de sodium et de trifluoroacétate de la trans 8-(aminométhyl)-2-(dimethylaminosulfamoyle)-4,8-dihydro-6-oxo (sulfooxy)-4,7-méthano-7H-pyrazolo[3,4-e] [1,3] diazépin-6(5H)-one

### Stade A

### trans [[2-(diméthylaminosulfamoyle)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

Sous azote, le dérivé obtenu au stade B de l'exemple 1 (200mg, 0.500mmol) est mis en solution dans le tétrahydrofurane anhydre (7mL). A -5°C, l'hydrure de sodium à 60% dans l'huile (30mg, 0.751mmol) est ajouté en une portion. Après 15 minutes, le chlorure de diméthylsulfamoyle (160µL, 1.502mmol) est additionné goutte à goutte. La température du mélange est remontée progressivement à l'ambiante. Après 3h d'agitation, le milieu est hydrolysé et extrait au dichlorométhane (10mL). La phase aqueuse est extraite au dichlorométhane (10mL). Les phases organiques réunies sont lavées par une solution aqueuse saturée de chlorure de sodium, séchées sur sulfate de sodium, concentrées sous pression réduite pour donner, après chromatographie sur colonne de silice (éluant gradient CH₂Cl₂/MeOH 100/0 à 98/2), le dérivé attendu (98mg, 0.193mmol, 39%) sous la forme d'une poudre blanche.
MS (ES(+)) : m/z [M+H]⁺ = 507
¹H NMR (300 MHz, CDCl₃) : δ (ppm) = 1.43 (s, 9H, C(CH₃)₃), 2.91 (s, 6H, N(CH₃)₂), 3.09 (dd, 1H, N-CH₂-CH-N), 3.32-3.44, 3.49-3.59 (m, 2H, CH-CH₂-NHBoc), 3.75 (m, 1H, N-CH₂-CH-N), 3.96 (d,1H, N-CH₂-CH-N), 4.63 (m, 1H, CH-CH₂-NHBoc), 4.95 (AB, 2H, CH₂-Ph), 5.12 (broad, 1H, NH), 7.36-7.55 (m, 5H, Ph), 7.78 (s, 1H, H pyrazole).

### Stade B

### Sel de pyridinium du trans [[2-(diméthylaminosulfamoyle)-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, le dérivé obtenu au stade précédent (90mg, 0.178mmol), le diméthylformamide (0.26mL), le dichlorométhane (0.79mL) et le palladium 10% sur charbon à 50% en eau (57mg, 0.027mmol) conduisent à l'intermédiaire débenzylé attendu.

L'intermédiaire débenzylé, la pyridine (0.59mL) et le complexe pyridine / sulfure de trioxyde (57mg, 0.356mmol) conduisent, après chromatographie sur colonne de silice (éluant gradient CH₂Cl₂/MeOH 100/0 à 80/20) au dérivé attendu (39mg, 0.067mmol, 38%).
MS (ES(-)) : m/z [M-H]⁻ = 495
¹H NMR (300MHz, MeOH-d₄): δ (ppm) = 1.46 (s, 9H, C(CH₃)₃), 2.91 (s, 6H, N(CH₃)₂), 3.30-3.59 (m, 5H, N-CH₂-CH-N, CH-CH₂-NHBoc, N-CH₂-CH-N), 4.60 (m, 1H, CH-CH₂-NHBoc), 4.92 (d,1H, N-CH₂-CH-N), 8.04 (m, 2H, Py), 8.14 (s, 1H, H pyrazole), 8.57(m, 1H, Py), 8.84 (m, 2H, Py).

### Stade C

### Sel de sodium du trans [[2-(diméthylaminosulfamoyle)-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade E de l'exemple 1, le sel obtenu au stade précédent (35mg, 0.061mmol) déposé dans un minimum de méthanol, la résine DOWEX 50WX8 (9 g) et la soude 2N (19mL) conduisent après élution à l'eau et lyophilisation au sel de sodium attendu (31mg, 0.060mmol, 100%) sous forme d'une poudre rosée
MS (ES(-)) : m/z [M-H]⁻ = 495
¹H NMR (300MHz, MeOH-d₆): δ (ppm) = 1.46 (s, 9H, C(CH₃)₃), 2.90 (s, 6H, C(CH₃)₂), 3.30-3.53 (m, 5H, N-CH₂-CH-N, CH-CH₂-NHBoc, N-CH₂-CH-N), 4.59 (m, 1H, CH-CH₂-NHBoc), 4.92 (d,1H, N-CH₂-CH-N), 8.14 (s, 1H, H pyrazole).

### Stade D

### Sel de sodium et de trifluoroacétate de la trans [[8-(aminométhyl)-2-(diméthylaminosulfamoyle)-4,8-dihydro-5-(sulfooxy)-4,7-méthano-7H-pyrazolo[3,4-e] [1,3] diazépin-6(5H)-one

En procédant comme indiqué au stade F de l'exemple 1, le sel de sodium obtenu au stade précédent (30mg, 0.058mmol), le dichlorométhane (0.8mL), l'acide trifluoroacétique (1.6 mL) dans le dichlorométhane (1.6 mL) conduisent au sel de sodium et de trifluoroacétate attendu (27mg, 0.051mmol, 88%) sous forme d'un solide beige..
MS (ES(-)) : m/z [M-H]⁻ = 396 ¹H NMR (300 MHz, DMSO-d₆) : δ (ppm) = 2.86(s, 6H, C(CH₃)₂), 3.33-3.45 (m, 5H, N-CH₂-CH-N, CH-CH₂-NHBoc, N-CH₂-CH-N), 4.71 (m, 1H, CH-CH₂-NHBoc), 4.85 (d,1H, N-CH₂-CH-N), 8.10 (broad, 3H, NH₃⁺) 8.34 (s, 1H, H pyrazole).

### Exemple 9 : Sel de sodium et de trifluoroacétate de la trans [[8-(aminométhyl)-1-(carbanimidoyl)-4,8-dihydro-6-oxo-5-(sulfooxy)-4,7-méthano-7H-pyrazolo[3,4-e] [1,3] diazépin-6(5H)-one

### Stade A

### trans [[1-[tert-butoxycarbonylamino-(tert-butoxycarbonyl imino)-méthyl]-4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyléthyle et

### trans [[2-[tert-butoxycarbonylamino-(tert-butoxycarbonyl imino)-méthyl]-4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyléthyle

Sous azote, le dérivé obtenu au stade B de l'exemple 1 (300mg, 0.751mmol) est mis en solution dans le dichlorométhane (1.25mL). La triéthylamine (523µL, 3.76mmol) et la N,N'-di-(tert-butoxycarbonyl)thiourée (415mg, 1.50mmol) sont ajoutées, suivies du chlorure de mercure (408mg, 1.50mmol). Après 20h d'agitation à température ambiante, la triéthylamine (261µL, 1.88mmol) et la N,N'-di-(tert-butoxycarbonyl)thiourée (208mg, 0.751mmol) sont ajoutées, suivies du chlorure de mercure (204mg, 0.751mmol). Après 44h d'agitation à température ambiante au total, le milieu est filtré sur membrane 0.45µm, rincé par le dichlorométhane (10mL) et le filtrat est concentré sous vide. Le résidu est chromatographié sur colonne de silice (éluant gradient CH₂Cl₂/acétate d'éthyle 100/0 à 85/15 par 5%) pour donner le dérivé attendu substitué en N1 (110mg, 0.171mmol, 23%) sous forme d'un solide beige, ainsi que le dérivé attendu substitué en N2 (137mg, 0.213mmol, 28%) sous forme d'un solide beige.
dérivé substitué en N1 :
MS (ES(+)) : m/z [M+H]⁺ = 642
¹H NMR (400 MHz, CDCl₃) : δ (ppm) = 1.44 (s, 9H, C(CH₃)₃), 1.48 (s, 9H, C(CH₃)₃), 1.55 (s, 9H, C(CH₃)₃), 3.35 (m, 2H, N-CH₂-CH-N), 3.68 (m, 1H, CH-CH₂-NHBoc), 3.83 (m, 1H, CH-CH₂-NHBoc), 3.93 (d, 1H, N-CH₂-CH-N), 4.92 (AB, 2H, CH₂-Ph), 5.02 (m, 1H, CH-CH₂-NHBoc), 5.38 (broad, 1H, NH), 7.41-7.44 (massif, 5H, Ph), 7.48 (s, 1H, H pyrazole), 8.93 (broad, 1H, NH).
dérivé substitué en N2 :

MS (ES(+)) : m/z [M+H]⁺ = 642
¹H NMR (400 MHz, CDCl₃) : δ (ppm) = 1.48 (s, 9H, C(CH₃)₃), 1.56 (s, 18H, 2 x C(CH₃)₃), 3.07 (d, 1H, N-CH₂-CH-N), 3.28 (m, 1H, CH-CH₂-NHBoc), 3.32 (dd, 1H, N-CH₂-CH-N), 3.86 (m, 1H, CH-CH₂-NHBoc), 3.90 (d,1H, N-CH₂-CH-N), 4.62 (m, 1H, CH-CH₂-NBoc), 4.92 (AB, 2H, CH₂-Ph), 5.19 (broad, 1H, NH), 7.41-7.44 (massif, 5H, Ph), 8.18 (s, 1H, H pyrazole), 8.70 (broad, 1H, NH).

### Stade B

### Sel de pyridinium du trans [[1-[tert-butoxycarbonylamino-(tert-butoxycarbonylimino)-méthyl]-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, le dérivé substitué en N1 obtenu au stade précédent (110mg, 0.171mmol), le diméthylformamide (0.49mL), le dichlorométhane (1.47mL) et le palladium 10% sur charbon à 50% en eau (55mg, 0.026mmol) conduisent à l'intermédiaire débenzylé attendu.

L'intermédiaire débenzylé, la pyridine (0.8mL) et le complexe pyridine / sulfure de trioxyde (55mg, 0.343mmol) conduisent, après chromatographie sur colonne de silice (éluant gradient CH₂Cl₂/MeOH 100/0 à 85/15 par 5%) au dérivé attendu (19mg, 0.026mmol, 16%) sous forme d'un solide beige.
MS (ES(+)) : m/z [M+H]⁺ = 632
¹H NMR (400MHz, MeOH-d₄): δ (ppm) = 1.50, 1.59 (s, 27H, 3x C(CH₃)₃), 3.51-3.57 (m, 3H, N-CH₂-CH-N, CH-CH₂-NHBoc), 3.96 (dd, 1H, N-CH₂-CH-N), 4.92 (signal H2O + N-CH₂-CH-N), (dd, 1H, CH-CH₂-NHBoc), 7.83 (s, 1H, H pyrazole)

### Stade C

### Sel de sodium et de trifluoroacétate de la trans-8-(aminométhyl)-1-(carbanimidoyl)-4,8-dihydro-5-(sulfooxy)-4,7-méthano-7H-pyrazolo[3,4-e][1,3]diazépin-6(5H)-one

En procédant comme indiqué au stade E de l'exemple 1, le sel obtenu au stade précédent (19mg, 0.027mmol), la résine DOWEX 50WX8 (2.3g) et la soude 2N (12mL) conduisent au sel de sodium attendu (17mg, 0.027mmol, 100%).

En procédant comme indiqué au stade F de l'exemple 1, le sel de sodium (17mg, 0.027mmol), le dichlorométhane (0.5mL), l'acide trifluoroacétique (1mL) dans le dichlorométhane (1mL) conduisent au sel de sodium et de trifluoroacétate attendu (10mg, 0.014mmol, 53%) sous forme d'un solide beige.
MS (ES (+)) : m/z [M+H]⁺= 335
¹H NMR (400 MHz, D₂O) sous forme de 2 conformères : δ (ppm) = 3.40-3.50, 3.54-3.63 (m, 4H, N-CH₂-CH-N et CH-CH₂-NH₃⁺), 4.82 (m, 1H, CH(B)-CH₂-NH₃⁺), 4.93 (d, 1H, N-CH₂-CH(B)-N), 4.99 (d, 1H, N-CH₂-CH(A)-N), 5.26 (m, 1H, CH(A)-CH₂-NH₃⁺), 7.74 (s, 1H, H(B) pyrazole), 8.00 (s, 1H, H(A) pyrazole)

### Exemple 10: Sel de sodium et de trifluoroacétate de la trans 8-(aminométhyl)-2-(carbanimidoyl)-4,8-dihydro-5-(sulfooxy)-4,7-méthano-7H-pyrazolo[3,4-e][1,3] diazépin-6(5H)-one

### Stade A

### Sel de pyridinium du trans [[2-[tert-butoxycarbonylamino-(tert-butoxycarbonylimino)-méthyl]-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade D de l'exemple 1, le dérivé substitué en N2 obtenu au stade A de l'exemple 9 (137mg, 0.213mmol), le diméthylformamide (0.61mL), le dichlorométhane (1.83mL) et le palladium 10% sur charbon à 50% en eau (55mg) conduisent à l'intermédiaire débenzylé attendu.

L'intermédiaire débenzylé, la pyridine (0.9mL) et le complexe pyridine / sulfure de trioxyde (68mg, 0.429mmol) conduisent, après chromatographie sur colonne de silice (éluant gradient CH₂Cl₂/MeOH 100/0 à 85/15 par 5%) au dérivé attendu (33mg, 0.046mmol, 22%) sous forme d'un solide beige.
MS (ES(-)) : m/z [M-H]⁻ = 630
¹H NMR (400MHz, MeOH-d₄): δ (ppm) = 1.50, 1.58 (s, 27H, C(CH₃)₃), 3.40-3.83 (m, 4H, N-CH₂-CH-N, CH-CH₂-NHBoc), 4.68 (dd, 1H, CH-CH₂-NHBoc), 5.00 (d, 1H, N-CH₂-CH-N), 7.50 (m, 2H, Py), 7.92 (m, 1H, Py), 8.37 (s, 1H, H pyrazole), 8.59 (m, 2H, Py)

### Stade B

### Sel de sodium et de trifluoroacétate de la trans 8-(aminométhyl)-2-(carbanimidoyl)-4,8-dihydro-5-(sulfooxy)-4,7-méthano-7H-pyrazolo[3,4-e][1,3] diazépin-6(5H)-one

En procédant comme indiqué au stade E de l'exemple 1, le sel obtenu au stade précédent (33mg, 0.046mmol), la résine DOWEX 50WX8 (4g) et la soude 2N (20mL) conduisent au sel de sodium attendu (30mg, 0.046mmol, 100%).

En procédant comme indiqué au stade F de l'exemple 1, le sel de sodium (30mg, 0.046mmol), le dichlorométhane (0.9mL), l'acide trifluoroacétique (1.7mL) dans le dichlorométhane (1.7mL) conduisent au sel de sodium et de trifluoroacétate attendu (24mg, 0.046mmol, 75%) sous forme d'un solide beige.
MS (ES(+)) : m/z [M+H]⁺ = 335
¹H NMR (400 MHz, D₂O) : δ (ppm) = 3.42-3.53, 3.69-3.76 (m, 4H, N-CH₂-CH-N et CH-CH₂-NH₃⁺), 4.90 (m, 1H, CH-CH₂-NH₃⁺), 5.04 (d, 1H, N-CH₂-CH-N), 8.39 (s, 1H, H pyrazole)

### Exemple 11 : sel de sodium et de trifluoroacétate de la trans 8-(amino-méthyl)-4,8-dihydro-2-(pipéridine-4-carbonyl)-5-(sulfooxy)-4,7-méthano-7H-pyrazolo[3,4-e][1,3] diazépin-6(5H)-one

### Stade A

### trans [[2-(1-tert-butoxycarbonyl-piperidine-4-carbonyl)-4,5,6,8-tétrahydro-6-oxo-5-(phénylméthoxy)-4,7-méthano-7H-pyrazolo[3,4-e][1,3] diazépin-8-yl]méthyl]-carbamate de 1,1-diméthyléthyle

L'acide N-boc-isonipecotique (0.137g, 0.6mmol) est mis en solution dans le diméthylformamide (3mL) en présence de diisopropyléthylamine (0.297mL, 1.8mmol) puis le tétrafluoroborate de O-(1H-benzotriazol-1-yl)-N,N,N',N'-tétraméthyluronium (0.211g, 0.6mmol) est ajouté suivi du pyrazole obtenu au stade B de l'exemple 1 (239mg, 0.6mmol) en solution dans le diméthylformamide (1mL). L'agitation est maintenue à température ambiante pendant 1h puis le mélange réactionnel est hydrolysé par l'eau (15mL) et extrait par l'acétate d'éthyle (30mL). La phase organique est lavée 5 fois par l'eau (15mL), séchée sur sulfate de magnésium puis concentrée sous vide. Le brut réactionnel est chromatographié sur colonne de silice (éluant cyclohexane/acétate d'éthyle 80/20 puis 70/30) pour conduire au dérivé attendu (0.219g, 0.36mmol, 60%) sous forme d'une huile.
MS (ES(+)) : m/z [M+H]⁺ = 611
¹H NMR (400 MHz, CDCl₃) : δ (ppm) = 1.47 (s, 9H, C(CH₃)₃), 1.48 (s, 9H, C(CH₃)₃), 1.56-1.97 (m, 4H, C(O)CH-CH₂-CH₂-N), 2.81-2.97 (m, 2H, CH₂-N(Boc)-CH₂), 3.09 (dd, 1H, N-CH₂-CH-N), 3.25-3.40 (m, 2H, N-CH₂-CH-N, C(O)CH(CH₂)-CH₂), 3.63 (m, 1H, CH₂-NHBoc), 3.89 (m, 1H, CH₂-NHBoc), 4.01 (d, 1H, N-CH₂-CH-N), 4.09-4.23 (m, 2H, CH₂-N(Boc)-CH₂), 4.63-4.72 (m, 1H, CH-CH₂-NHBoc), 4.95 (AB, 2H, CH₂-Ph), 5.11 (broad, 1H, NH), 7.32-7.50 (m, 5H, H aromatiques), 7.44 (s, 1H, H pyrazole).

### Stade B

### Sel de pyridinium de la trans [[2-(1-tert-butoxycarbonyl-piperidine-4-carbonyl)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade D de l'exemple 1, le dérivé obtenu au stade A précédent (0.214g, 0.35mmol), un mélange diméthylformamide/CH₂Cl₂ 1/3 anhydre (2mL) et le palladium 10% sur charbon à 50% en eau (111mg) conduisent à l'intermédiaire débenzylé attendu.

L'intermédiaire débenzylé, la pyridine (1mL) et le complexe pyridine / sulfure de trioxyde (0.111mg, 0.70mmol) conduisent, après chromatographie sur colonne de silice (éluant gradient CH₂Cl₂/MeOH 100/0 à 85/15) au dérivé attendu (0.061g, 0.090mmol, 34%) sous la forme d'un amorphe.
¹H NMR (400 MHz, MeOH-d₄) : δ (ppm) = 1.29 (s, 9H, C(CH₃)₃), 1.32 (s, 9H, C(CH₃)₃), 1.50 (m, 2H, C(O)CH-CH₂-CH₂), 1.80 (m, 2H, C(O)CH-CH₂-CH₂), 2.79 (m, 2H, CH₂-N(Boc)-CH₂), 3.20-3.46 (m, 4H, CH-CH₂-NHBoc, N-CH₂-CH-N), 3.62 (m, 1H, C(O)CH-CH₂), 3.95 (m, 2H, CH₂-N(Boc)-CH₂), 4.46 (m, 1H, CH-CH₂-NHBoc), 4.78 (d, 1H, N-CH₂-CH-N), 8.17 (s, 1H, H pyrazole).

### Stade C

### Sel de sodium de la trans [[2-(1-tert-butoxycarbonyl-piperidine-4-carbonyl)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyléthyle

En procédant comme indiqué au stade E de l'exemple 1, le dérivé obtenu au stade précédent (61mg, 0.09mmol), la résine DOWEX 50WX8 (9.4g) et la soude 2N (50mL) conduisent au dérivé attendu (55mg, 0.088mmol, 100%) sous la forme d'un amorphe.
MS (ES(-)) : m/z [M-H]⁻ = 599
¹H NMR (400 MHz, DMSO-d₆) : δ (ppm) = 1.45 (s, 9H, C(CH₃)₃), 1.48 (s, 9H, C(CH₃)₃), 1.53 (m, 2H, C(O)CH-CH₂-CH₂), 1.87 (m, 2H, C(O)CH-CH₂-CH₂), 2.91 (m, 2H, CH₂-N(Boc)-CH₂), 3.25-3.44 (m, 4H, CH-CH₂-NHBoc, N-CH₂-CH-N), 3.63 (m, 1H, C(O)CH-CH₂), 3.92 (m, 2H, CH₂-N(Boc)-CH₂), 4.44 (m, 1H, CH-CH₂-NHBoc), 4.81 (d, 1H, N-CH₂-CH-N), 7.09 (broad, 1H, NH), 8.17 (s, 1H, H pyrazole).

### Stade D

### Sel de sodium et de trifluoroacétate de la trans-8-(amino-méthyl)-4,8-dihydro-2-(pipéridine-4-carbonyl)-5-(sulfooxy)-4,7-méthano-7H-pyrazolo[3,4-e][1,3] diazépin-6(5H)-one

En procédant comme indiqué au stade F de l'exemple 1, le sel de sodium (55mg, 0.088mmol), le dichlorométhane (2.28mL), l'acide trifluoroacétique (1.14mL) dans le dichlorométhane (1.14mL) conduisent au sel de sodium et de trifluoroacétate attendu (53mg, 0.082mmol, 93%) sous la forme d'un amorphe jaune.
MS (ES(-)) : m/z [M-H]⁻ = 398
¹H NMR (400 MHz, DMSO-d₆) : δ (ppm) = 1.66 (m, 2H, CH₂-NH-CH₂), 1.95 (m, 2H, CH₂-NH-CH₂), 2.44-2.62, 2.85-3.00 et 3.17-3.42 (m, 9H, CH-CH₂-NH;⁺, N-CH₂-CH-N, C(O)CH(CH₂)-CH₂ et CH₂-NH-CH₂), 4.65 (dd, 1H, CH-CH₂-NH;⁺), 4.77 (d, 1H, N-CH₂-CH-N), 7.78 (s, 1H, H pyrazole), 8.00 (broad, 5H, NH₃⁺, NH₂⁺).

### Exemple 12 : Le sel de sodium et de trifluoroacétate de la trans 8-(amino-méthyl)-2-(3-amino-3-carboxy-propyl)-4,8-dihydro-5-(sulfooxy)-4,7-méthano-7H-pyrazolo[3,4-e][1,3] diazépin-6(5H)-one

### Stade A

### trans [[2-(3-tert-butoxycarbonyl-3-tert-butoxycarbonylamino-propyl)-4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyle

Le composé obtenu au stade B de l'exemple 1 (200mg, 0.50mmol) est mis en solution dans du diméthylformamide anhydre (3mL) en présence de boc-L-glutamic acide 1-tert-butyl ester (159mg, 0.525mmol) et d'hydrate de 1-hydroxybenzotriazole (85mg, 0.63mmol) puis du chlorhydrate de N-(3-diméthylaminopropyl)-N'-éthylcarbodiimide (101mg, 0.525mmol) est ajouté après refroidissement à 0°C. Le mélange réactionnel est agité à température ambiante pendant une nuit. Après dilution avec l'acétate d'éthyle, le mélange est lavé successivement avec une solution aqueuse 10% d'acide tartrique, une solution aqueuse saturée de NaHCO₃, H₂O, puis une solution aqueuse saturée de chlorure de sodium. La phase organique est séchée sur sulfate de sodium, filtrée puis concentrée sous vide. Le brut ainsi obtenu est purifié par chromatographie sur silice (éluant CH₂Cl₂/AcOEt 90/10) pour donner le produit attendu (92mg, 0.134, 27%)
MS (ES(+)) : m/z [M+H]⁺ = 685

### Stade B

### Sel de pyridinium du trans [[2-(3-tert-butoxycarbonyl-3-tert-butoxycarbonylamino-propyl)-4,5,6,8-tétrahydro-6-oxo-5-(sulfooxy)-4,7-méthano-7H-pyrazolo[3,4-e][1,3] diazépin-8-yl]méthyl]-carbamate de 1,1-diméthyle

En procédant comme indiqué au stade D de l'exemple 1, le dérivé obtenu au stade précédent (92mg, 0.13mmol), un mélange diméthylformamide/CH₂Cl₂ 1/3 anhydre (0.8mL) et le palladium 10% sur charbon à 50% en eau (43mg) conduisent à l'intermédiaire débenzylé attendu.

L'intermédiaire débenzylé, la pyridine (380µL) et le complexe pyridine / sulfure de trioxyde (43mg, 0.27mmol) conduisent, après chromatographie sur colonne de silice (éluant CH₂Cl₂/MeOH 90/10) au sel attendu (28mg, 0.037mmol, 19%).
MS (ES(+)) : m/z [M+H]⁺ = 675
¹H RMN (400MHz, MeOH-d₄) : δ(ppm) = 1.52 (m, 27H, 3x C(CH₃)₃), 2.05 (m, 2H, C(O)CH₂-CH₂-CH-(N)CO₂t-Bu), 2.25 (m, 2H, C(O)CH₂-CH₂-CH-(N)CO₂t-Bu), 3.50 (m, 4H, N-CH₂-CH-N et CH-CH₂-NHBoc), 4.11 (m, 1H, C(O)CH₂-CH₂-CH-(N)CO₂t-Bu), 4.62(dd, 1H, CH-CH₂-NHBoc), 5.05 (d, 1H, N-CH₂-CH-N), 8.42 (s, 1H, H pyrazole).

### Stade C

Sel de sodium et de trifluoroacétate de *trans* 8-(amino-méthyl)-2-(3-amino-3-carboxy-propyl)-4,8-dihydro-5-(sulfooxy)-4,7-méthano-7*H-*pyrazolo[3,4-e][1,3] diazépin-6(5H)-one En procédant comme indiqué au stade E de l'exemple 1, le dérivé obtenu au stade précédent (28mg, 0.04mmol), la résine DOWEX 50WX8 (3.5g) et la soude 2N (17.5mL) conduisent au sel de sodium attendu.

En procédant comme indiqué au stade F de l'exemple 1, le sel de sodium (17mg, 0.027mmol), le dichlorométhane (0.86mL), l'acide trifluoroacétique (0.86mL) dans le dichlorométhane (0.86mL) conduisent au sel de sodium et de trifluoroacétate attendu (27mg, 0.049mmol, 100%) sous forme d'une gomme jaune.
MS (ES(-)) : m/z [M-H]⁻ = 417
¹H RMN (400MHz, MeOH-d₄) : δ(ppm) = 2.21-2.44 (m, 5H, C(O)CH₂-CH₂-CH-(N)CO₂t-Bu, C(O)CH₂-CH₂-CH-(N)CO₂t-Bu), 3.36-3.54 (m, 4H, N-CH₂-CH-N et CH-CH₂-NHBoc), 4.11 (m, 1H, C(O)CH₂-CH₂-CH-(N)CO₂t-Bu), 4.82 (dd, 1H, CH-CH₂-NHBoc), 4.98 (d, 1H, N-CH₂-CH-N), 7.79 (s, 1H, H pyrazole).

### Exemple 13 : sel de sodium et de trifluoroacétate de la trans 8-(guanidino-méthyl)-4,8-dihydro-5-(sulfooxy)-4,7-méthano-7H-pyrazolo[3,4-e][1,3] diazépin-6(5H)-one

### Stade A

### trans [[[4,5,6,8-tétrahydro-6-oxo-5-(phénylméthoxy)-4,7-méthano-7H-pyrazolo[3,4-e][1,3] diazépin-8-yl]méthyl]-carbonimidoyl]carbamate de bis-bis(1,1-diméthyléthyle)

Sous azote, l'azoture intermédiaire obtenu au stade B de l'exemple 1 (1.5g, 4.6mmol) est mis en solution dans le tétrahydrofurane (29mL) puis la solution est refroidie à 0°C. La triméthylphosphine (1M dans le tétrahydrofurane, 4.6mL, 4.6mmol) est ajoutée. La solution est agitée 2h à température ambiante, puis la 1,3-bis-(Boc)-2-méthyl-2-thiopseudourée (1.34g, 4.6mmol) est ajoutée. Après une nuit d'agitation à température ambiante, de l'eau (0.83mL, 46mmol) est ajoutée, puis le mélange est conservé 16h à 5°C. Celui-ci est concentré à sec, puis purifié par chromatographie sur colonne de silice (éluant cyclohexane/acétate d'éthyle, gradient de 80/20 à 0/100) pour donner le produit attendu (560 mg, 1.03mmol, 22%).
MS (ES(+)) : m/z [M+H]⁺ = 542
¹H NMR (400 MHz, CDCl₃) : δ (ppm) = 1.48 (s, 9H, C(CH₃)₃), 1.54 (s, 9H, C(CH₃)₃), 3.10 (d, 1H, N-CH₂-CH-N), 3.36 (dd,1H, N-CH₂-CH-N, 3.70-3.80 (m, 1H, CH-CH₂-NH-C=NBoc), 4.00 (d, 1H, N-CH₂-CH-N), 4.13-4.25(m, 1H CH-CH₂-NH-C=NBoc), 4.68 (dd, 1H, CH-CH₂-NH-C=NBoc), 4.93 (AB, 2H, CH₂-Ph), 7.28-7.44 (m, 6H, H pyrazole + Ph), 9.38 (broad, 1H, NH), 11.35 (broad, 1H, NH).

### Stade B

### trans [[[1-tert-butoxycarbamate-4,5,6,8-tétrahydro-6-oxo-5-(phénylméthoxy)-4,7-méthano-7H-pyrazolo[3,4-e][1,3] diazépin-8-yl]méthyl]-carbonimidoyl]carbamate de bis-bis(1,1-diméthyléthyle)

Sous azote, le composé obtenu au stade précédent (130mg, 0.24mmol) est mis en solution dans le dichlorométhane (12mL). La N,N-diméthylaminopyridine (15mg, 0.12mmol) est ajoutée, suivie du di-tert-butyl dicarbonate (105mg, 0.48mmol). Après une nuit d'agitation à température ambiante, le milieu est hydrolysé par une solution aqueuse d'acide tartrique 10% (10mL). Après 10min d'agitation, les phases sont séparées, la phase aqueuse est extraite par le dichlorométhane (10mL). Les phases organiques, sont rassemblées, lavées à l'eau puis par une solution saturée NaCl, séchées sur MgSO₄ et concentrées à sec. Le produit brut est purifié sur colonne de silice (éluant cyclohexane/acétate d'éthyle gradient de 80/20 à 0/100) pour fournir le produit attendu (100mg, 0.16mmol, 65%).
MS (ES(+)) : m/z [M⁺] = 642
¹H NMR (400 MHz, CDCl₃) : δ (ppm) = 1.43 (s, 9H, C(CH₃)₃), 1.46 (s, 9H, C(CH₃)₃), 1.54 (s, 9H, C(CH₃)₃), 3.14-3.36 (m, 2H, N-CH₂-CH-N), 3.87-3.93 (m, 2H, CH-CH₂-NH-C=Nboc, N-CH₂-CH-N), 4.16 (m, 1H CH-CH₂-NH-C=NBoc), 4.68 (dd, 1H, CH-CH₂-NH-C=NBoc), 4.79 (d, 1H, CH₂-Ph), 4.93 (d, 1H, CH₂-Ph), 7.32-7.35 (m, 5H, Ph), 7.83 (s, 1H, H pyazole), 8.90 (broad, 1H, NH), 11.38 (broad, 1H, NH).

### Stade C

### sel de sodium et de trifluoroacétate de la trans-8-(guanidino-méthyl)-4,8-dihydro-5-(sulfooxy)-4,7-méthano-7H-pyrazolo[3,4-e] [1,3] diazépin-6(5H)-one

En procédant comme indiqué au stade D de l'exemple 1, le dérivé obtenu au stade précédent (88mg 0.14mmol), un mélange diméthylformamide/CH₂Cl₂ 1/3 anhydre (0.8mL) et le palladium 10% sur charbon à 50% en eau (44mg) conduisent à l'intermédiaire débenzylé attendu.

L'intermédiaire débenzylé, la pyridine (0.4mL) et le complexe pyridine / sulfure de trioxyde (43mg, 0.272 mmol) conduisent, après chromatographie sur colonne de silice (éluant CH₂Cl₂/méthanol, gradient de 100/0 à 80/20) au composé attendu (55mg, 0.077mmol, 56%).
MS (ES(+)) : m/z [M+H]⁺ = 632

En procédant comme indiqué au stade E de l'exemple 1, le sel obtenu au stade précédent (50mg, 0.07mmol), la résine DOWEX 50WX8 (3.5g) et la soude 2N (17.5mL) conduisent au sel de sodium attendu (42mg, 0.064mmol, 91%) sous forme d'un lyophilisat blanc.
MS (ES(-)) : m/z [M-H]⁻ = 630

En procédant comme indiqué au stade F de l'exemple 1, le sel de sodium (42mg, 0.064mmol, 91%), le dichlorométhane (3mL), l'acide trifluoroacétique (2mL) dans le dichlorométhane (2mL) conduisent au sel de sodium et de trifluoroacétate attendu (40mg, 0.058mmol, 95%) sous forme d'une poudre beige.
MS (ES(-)) : m/z [M-H]⁻ = 330
¹H NMR (400 MHz, D₂O) : δ (ppm) = 3.43-3.70 (m, 5H, N-CH₂-CH-N, CH-CH₂-NH-C=Nboc, CH-CH₂-NH-C=NBoc), 4.92 (d, 1H, N-CH₂-CH-N), 7.73 (s, 1H, H pyrazole) ¹⁹F NMR (300 MHz, DMSO-d₆) : δ (ppm)= -74.17 (s, CF3)

### Exemple 14 : Sel de sodium et de trifluoroacétate de la trans 8-(guanidino-méthyl)-4,8-dihydro-1-méthyl-5-(sulfooxy)-4,7-méthano-7H-pyrazolo[3,4-e][1,3] diazépin-6(5H)-one

### Stade A

### 4,7-dihydro-1-méthyl-4-((phénylméthoxy)amino)-1H-pyrazolo [3,4-c]pyridine-6(5H),7-dicarboxylate de 6-(1,1-diméthyléthyle) et de 7-méthyle

Le dérivé (4,7-dihydro-4-hydroxy-1-methyl-1H-pyrazolo[3,4-c]pyridine-6(5*H*),7-dicarboxylate de 6-(1,1-diméthyléthyle) et de 7-méthyle, décrit dans la demande WO 02/100860 (stade C, exemple 18) (10g, 32.12mmol) est mis en suspension dans le dichlorométhane (100mL) à température ambiante sous azote et sous agitation. La suspension se dissout après l'ajout de triéthylamine (14.30mL, 10.28mmol). Au milieu réactionnel refroidi à -78°C est additionné goutte à goutte une solution de chlorure de méthane sulfonyle (11.4mL, 96.36mmol) dans le dichlorométhane (12mL). Après 30min d'agitation, l'alcool est complètement transformé en mésylate.

Une solution de *O*-benzyl-hydroxylamine dans le dichlorométhane est fraîchement préparée à partir du chlorhydrate de *O*-benzylhydroxylamine (25.4g, 160.6 mmol). Le chlorhydrate de *O-*benzylhydroxylamine est dissout dans un mélange de dichlorométhane (100mL) et d'eau (50mL). Une solution de soude 2N (85mL, 176.66mmol) est ajoutée à 0°C. Après 10 min de contact et décantation, la phase organique est séchée sur sulfate de magnésium durant 45min, puis concentrée à demi volume. L'addition de cette solution au mésylate préparé ci-dessus se fait à -78°C goutte à goutte sur 1h. Le mélange réactionnel est agité en laissant la température remonter progressivement à température ambiante. Le milieu est traité par addition d'eau (200mL) et dilué avec du dichlorométhane (100mL). Après agitation et décantation, la phase aqueuse est extraite au dichlorométhane. La phase organique est lavée avec une solution de NaCl saturée (200mL), séchée, puis concentrée à sec pour donner une poudre amorphe blanche, qui après chromatographie conduit au dérivé attendu (8.25g, 21.2mmol, 66%).
MS (ES(+)) : m/z [M+H]⁺ = 417
¹H NMR (400MHz, CDCl₃): Description d'un des deux diastéréoisomères (sous forme de 2 rotamères) δ (ppm) = 1.43 (s, 9H, C(CH₃)₃), 3.15 (dd, 1H, N-CH₂-CH-N), 3.68 / 3.70 (s, 3H, CH₃), 3.84(s, 3H, CH₃), 3.98 (m, 2H, N-CH₂-CH-N), 4.6-4.8 (massif, 3H, NH-O-CH₂-Ph et N-CH₂-CH-N), 5.40 / 5.8 (s, 1H, CH-CO₂Me), 7.22-7.31(massif, 5H, Ph), 7.40(s, 1H, H pyrazole)

### Stade B

### trans 1-méthyl-6-oxo-5-(phénylméthoxy)-4,5,6,8-tétrahydro-4,7-méthano-1H-pyrazolo[3,4-e] [1,3]diazépine-8 (7H) carboxylate de méthyle

Une solution 4N de HCl/ dioxane (400mL) est coulée sur une solution du dérivé obtenu au stade précédent (21g, 50.42mmol) dissout dans le dioxane (50mL) à température ambiante. Le mélange réactionnel est agité pendant 30min, puis le dioxane est évaporé. Le résidu est repris sous agitation dans un mélange d'eau (100mL) et d'acétate d'éthyle (500mL). Une solution d'ammoniaque concentrée à 20% (42mL) est ajoutée à 0°C. L'agitation est poursuivie pendant 30min. Après décantation la phase aqueuse est ré-extraite avec de l'acétate d'éthyle (2x300mL), la dernière extraction étant réalisée après saturation de la phase aqueuse avec du NaCl. La phase organique est séchée puis concentrée. On obtient l'intermédiaire pipéridine déprotégée sous forme d'une huile jaune (15.7g, 49.4mmol, 98%) qui est reprise dans l'acétonitrile (400mL). A ce mélange refroidi à 0°C, sont ajoutés la triéthylamine (21mL, 151.2mmol), puis le diphosgène (3.04mL, 25.2mmol) coulé goutte à goutte sur 30min. Après une nuit d'agitation à température ambiante, le milieu est concentré puis repris avec de l'acétate d'éthyle (500mL) et traité avec une solution d'acide tartrique 10% (200mL). Le mélange est agité, décanté. La phase organique est lavée par une solution d'acide tartrique 10% (2*200mL), par une solution de NaCl saturée, puis séchée et concentrée sous pression réduite. Le produit blanc obtenu (15.3g, 44.0mmol, 89%) est repris dans le dichlorométhane (150mL). Du 1-8-Diazabicyclo[5.4.0]undéc-7-ène (7.53mL,50.04mmol) est ajouté goutte à goutte. Le mélange est agité pendant 2h, traité avec de l'eau (200mL), agité, décanté. La phase organique est lavée avec de l'eau (2*200mL), puis avec une solution de NaCl saturée (1*200mL), séchée sur MgSO₄, puis concentrée à sec, pour donner le dérivé attendu (14.72g, 37.4mmol, 85%), sous forme d'un solide blanc.
MS (ES(+)) : m/z [M+H]⁺ = 343
¹H NMR (400MHz, CDCl₃): δ (ppm) = 3.25 (d, 1H, N-CH₂-CH-N), 3.45 (d, 1H, N-CH₂-CH-N), 3.80 (s, 3H, CH₃), 3.88 (s, 3H, CH₃), 3.9 (s, 1H, N-CH₂-CH-N), 4.7 (d, 1H, N-O-CH₂-Ph), 5.02 (d, 1H, N-O-CH₂-Ph), 5.22 (s, 1H, CH-CO₂Me), 7.39-7.43(massif, 6H, H pyrazole + Ph)

### Stade C

### trans 4,8-dihydro-8-(hydroxyméthyl)-1-méthyl-5-(phénylméthoxy)-4,7-méthano-7H-pyrazolo[3,4-e] [1,3] diazépin-6(5H)-one

Une solution de l'urée obtenue au stade précédent (5g, 14.60mmol) dans un mélange de tétrahydrofurane (150mL) / méthanol (50mL) anhydres, sous azote et sous agitation, est refroidie à -10°C. Du borohydrure de lithium (668mg, 30.67mmol) est ajouté au milieu réactionnel. Après 2h d'agitation à -10°C, 1.2éq. supplémentaires de LiBH₄ sont ajoutés. La réaction est traitée à froid 2h plus tard par une solution à 10% de NaH₂PO₄. Le tétrahydrofurane et le méthanol sont évaporés sous pression réduite (200 mbar, 40°C). Le mélange résiduel est repris avec de l'acétate d'éthyle (200mL), agité et décanté. La phase aqueuse est réextraite avec de l'acétate d'éthyle (100mL). La phase organique est séchée sur sulfate de magnésium puis concentrée à sec. La poudre jaune clair obtenue (6.6g) est chromatographiée sur silice (éluant-acétate d'éthyle) pour donner le dérivé attendu (3.2g, 10.18mmol, 64%).
MS (ES(+)) : m/z [M⁺] = 315
¹H NMR (400 MHz, DMSO-*d₆*): δ (ppm) = 3.16 (dd, 1H, N-CH₂-CH-N), 3.48 (d, 1H, N-CH₂-CH-N), 3.71 (s, 3H, CH₃), 3.81-3.91 (massif, 2H, CH₂OH), 4.44 (m, 1H, N-CH₂-CH-N), 4.48 (m, 1H, CH-CH₂OH), 4.88 (m, 2H, N-O-CH₂-Ph), 5.20 (m, 1H, OH), 7.35-7.40 (massif, 6H, H pyrazole + Ph).

### Stade D

### trans 4,8-dihydro-1-méthyl-8-[(méthylsulfonyl)oxyméthyl)]-5-(phénylméthoxy)-4,7-méthano-7H-pyrazolo[3,4-e][1,3] diazépin-6(5H)-one

L'alcool obtenu au stade précédent (2.76g, 8.78mmol) est mis en solution dans du dichlorométhane (100mL) à température ambiante sous azote et sous agitation. Après refroidissement à 0°C, sont ajoutés de la triéthylamine (1.83mL, 13.17mmol), puis goutte à goutte une solution de chlorure de méthanesulfonyl (1.61g, 14.05mmol) dans le dichlorométhane (100mL). Le bain de glace est retiré en fin d'addition. Au bout d'une heure d'agitation à température ambiante, la réaction est traitée sous agitation par une solution à 10% de NaH₂PO₄ (80mL). La phase aqueuse est réextraite au dichlorométhane (50mL). Les phases organiques sont rassemblées, séchées, puis concentrées sous pression réduite pour donner le dérivé attendu (3.44g, 8.78mmol, rendement quantitatif).
MS (ES(+)) : m/z [M+H]⁺ = 393
¹H NMR (400 MHz, DMSO-*d₆*): δ (ppm) = 3.23 (dd, 1H, N-CH₂-CH-N), 3.26 (s, 3H, CH₃), 3.45 (d, 1H, N-CH₂-CH-N), 3.76 (s, 3H, CH₃), 4.52 (m, 1H, N-CH₂-CH-N), 4.58 (dd, 1H, CH-CH₂-OMs), 4.66 (dd, 1H, CH-CH₂-OMs), 4.88 (m, 3H, CH-CH₂-OMs et N-O-CH₂-Ph), 7.35-7.45 (massif, 6H, H pyrazole + Ph)

### Stade E

### Trans 8-(azidométhyl)-4,8-dihydro-1-méthyl-5-(phénylméthoxy)-4,7-méthano-7H-pyrazolo[3,4-e] [1,3] diazépin-6(5H)-one

De l'azidure de sodium est ajouté en une seule fois (1.71g, 26.3mmol) à une solution du dérivé mésylé obtenu au stade D de l'exemple 9 (3.44g, 8.78mmol) dans le diméthylformamide (70mL) à température ambiante sous azote et sous agitation. Le milieu réactionnel est chauffé à 65°C une nuit, puis traité par une solution aqueuse à 10% de NaH₂PO₄ (50mL). La phase aqueuse est extraite au dichlorométhane (2*50mL). La phase organique est séchée, puis concentrée sous pression réduite pour donner 3.96g de dérivé attendu (3g, 8.78mmol, 100%).
MS (ES(+)) : m/z [M⁺] = 340
¹H NMR (400 MHz, DMSO-*d₆*): δ (ppm) = 3.20 (dd, 1H, N-CH₂-CH-N), 3.48 (d, 1H, N-CH₂-CH-N), 3.66 (dd, 1H, CH-CH₂-N₃), 3.72 (s, 3H, CH₃), 3.92 (dd, 1H, CH-CH₂-N₃), 4.50(d, 1H, N-CH₂-CH-N), 4.76 (dd, 1H, CH-CH₂-N₃), 4.89 (m, 2H, N-O-CH₂-Ph), 7.35-7.45 (massif, 6H, H pyrazole + Ph)

### Stade F

### trans [[[4,5,6,8-tétrahydro-1-méthyl-6-oxo-5-(phénylméthoxy)-4,7-méthano-7H-pyrazolo[3,4-e] [1,3] diazépin-8-yl]méthyl]-carbonimidoyl]carbamate de bis-bis(1,1-diméthyle)

La triméthylphosphine (1M dans le tétrahydrofurane, 0.46mL, 0.46mmol) est ajoutée goutte à goutte à une solution du dérivé obtenu au stade précédent (150mg, 0.44mmol) dans le tétrahydrofurane (2mL) à 0°C sous azote. Après 3h d'agitation à température ambiante, la 1,3-bis(*tert*-butoxycarbonyl)-2-méthyl-2-thiopseudourée (141mg, 0.48mmol) est ajoutée au milieu réactionnel. Après une nuit d'agitation à température ambiante, l'eau (0.5mL) est ajoutée et le milieu est concentré sous pression réduite pour donner une poudre jaune pâle, qui, après purification par chromatographie sur colonne de silice (éluant cyclohexane/acétate d'éthyle 5/5), conduit au produit attendu (152mg, 0.273mmol, 61%).
MS (ES(+)) : m/z [M+H]⁺ = 556, [[M-(BOC)]⁺] = 456, [[M-(2BOC)]⁺] = 356
¹H NMR (400MHz, DMSO-*d₆*): δ (ppm) = 1.40 (s, 9H, C(CH₃)₃), 1.50 (s, 9H, C(CH₃)₃), 3.20 (dd, 1H, N-CH₂-CH-N), 3.35 (d, 1H, N-CH₂-CH-N), 3.68 (m, 1H, CH-CH₂-NH), 3.79 (m, 1H, N-CH₂-CH-N), 3.86 (s, 3H, CH₃), 4.50 (d, 1H, N-CH₂-CH-N), 4.70 (m, 1H, CH-CH₂-NH), 4.90 (m, 2H, CH₂-Ph), 7.35-7.41 (m, 6H, Ph + H pyrazole), 8.68 (broad, 1H, NH)

### Stade G

### Sel de pyridinium du trans [[[4,5,6,8-tétrahydro-1-méthyl-6-oxo-5-(sulfooxy)-4,7-méthano-7H-pyrazolo[3,4-e][1,3] diazépin-8-yl]méthyl]-carbonimidoyl]carbamate de bis-bis(1,1-diméthyle)

Sous azote, du palladium 10% sur charbon (100mg) est ajouté à une solution du composé obtenu au stade précédent (142mg, 0.255mmol) dans le méthanol (5mL). Après trois purges vide/azote, le milieu réactionnel est hydrogéné à pression atmosphérique pendant 3h. Le méthanol est concentré sous vide pour donner l'intermédiaire débenzylé.

L'intermédiaire débenzylé est repris dans la pyridine (1mL) en présence du complexe pyridine / sulfure de trioxyde (82mg, 0.511mol). Après une nuit d'agitation à température ambiante, le milieu est concentré sous vide. Le brut réactionnel est chromatographié sur colonne de silice (éluant gradient CH₂Cl₂/méthanol 100/0 à 80/20) pour donner le dérivé attendu (62mg, 0.11mmol, 45%).
MS (ES(+)) : m/z [M+H] ⁺ = 546
¹H NMR (400 MHz, DMSO-*d₆*) : δ (ppm) = 1.42 (s, 9H, C(CH₃)₃), 1.51 (s, 9H, C(CH₃)₃), 3.28 (dd, 1H, N-CH₂-CH-N), 3.36 (d, 1H, N-CH₂-CH-N), 3.70 (m, 1H, CH-CH₂-NH), 3.80 (m, 1H, CH-CH₂-NH), 3.87 (s, 3H, CH₃), 4.68 (m, 2H, N-CH₂-CH-N, CH-CH₂-NH), 7.38 (s, 1H, H pyrazole), 8.90 (broad, 1H, NH)

### Stade H

### Sel de sodium de la trans [[[4,5,6,8-tétrahydro-1-méthyl-6-oxo-5-(sulfooxy)-4,7-méthano-7H-pyrazolo[3,4-e] [1,3] diazépin-8-yl]méthyl]-carbonimidoyl]carbamate de bis-bis(1,1-diméthyle)

En procédant comme indiqué au stade E de l'exemple 1, le dérivé obtenu au stade précédent (62mg, 0.11mmol), la résine DOWEX 50WX8 (62g) et la soude 2N (300mL) conduisent au dérivé attendu (57mg, 0.11mmol, 100%) sous forme d'une poudre blanche amorphe.
MS (ES (+)) : m/z [M+H]⁺ = 546
¹H NMR (400 MHz, DMSO-*d*₆) : δ (ppm) = 1.42 (s, 9H, C(CH₃)₃), 1.51 (s, 9H, C(CH₃)₃), 3.27-3.34 (m, 2H, N-CH₂-CH-N), 3.70 (m, 1H, CH-CH₂-NH), 3.80 (m, 1H, CH-CH₂-NH), 3.87 (s, 3H, CH₃), 4.68 (m, 2H, N-CH₂-CH-N, CH-CH₂-NH), 7.36 (s, 1H, H pyrazole), 8.78 (broad, 1H, NH), 11.42 (broad, 1H, NH)

### Stade I

### Sel de sodium et de trifluoroacétate de la trans 8-(guanidino-méthyl)-4,8-dihydro-1-méthyl-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (57mg, 0.11mmol), le dichlorométhane (7mL), l'acide trifluoroacétique (3mL) dans le dichlorométhane (3mL) conduisent au sel de sodium et de trifluoroacétate attendu (56mg, 0.11mmol, 100%).
MS (ES(-)) : m/z [M-H]⁻ = 344
¹H NMR (400 MHz, DMSO-*d*₆) : δ (ppm) = 3.28 (m, 1H, N-CH₂-CH-N), 3.42 (m, 1H, N-CH₂-CH-N), 3.55 (m, 1H, CH-CH₂-NH), 3.69 (m, 1H, CH-CH₂-NH), 3.78 (s, 3H, CH₃), 4.69-4.72 (m, 2H, N-CH₂-CH-N, CH-CH₂-NH), 7.0-7.35 (m, 3H, NH₃⁺), 7.40 (s, 1H, H pyrazole), 7.45 (broad, 1H, NH)

### Exemple 15 : Sel de sodium et de trifluoroacétate de la trans 8-(guanidino-méthyl)-1-éthylcarbamoyl-4,8-dihydro-5-(sulfooxy)-4,7-méthano-7H-pyrazolo[3,4-e][1,3]diazépin-6(5H)-one

### Stade A

### trans [[[1-éthylcarbamoyl-4,5,6,8-tétrahydro-6-oxo-5-(phénylméthoxy)-4,7-méthano-7H-pyrazolo[3,4-e] [1,3] diazépin-8-yl]méthyl]-carbonimidoyl]-carbamate de bis-bis(1,1-diméthyléthyle) et

### trans [[[2-éthylcarbamoyl-4,5,6,8-tétrahydro-6-oxo-5-(phénylméthoxy)-4,7-méthano-7H-pyrazolo[3,4-e] [1,3] diazépin-8-yl]méthyl]-carbonimidoyl]-carbamate de bis-bis(1,1-diméthyléthyle)

Le dérivé obtenu au stade A de l'exemple 13 (0.3g, 0.55mmol) est mis en solution dans un mélange acétonitrile/ tétrahydrofurane anhydres 8/2 (10mL), sous azote. La solution est refroidie par un bain eau-glace. L'isocyanate d'éthyle (0.050mL, 0.609mmol) est ensuite ajouté goutte à goutte. Après quelques minutes d'agitation, le bain eau-glace est enlevé. Le mélange réactionnel est agité à température ambiante pendant une nuit. De l'eau (0.3mL, 0.017mmol) est ajoutée, suivi de l'éthanol (0.3mL). Le mélange est agité pendant 30mn puis concentré sous vide. Le mélange épais obtenu est repris dans l'eau (5mL), puis du dichlorométhane (10mL) est rajouté. Après une bonne agitation et décantation, la phase aqueuse est extraite par le dichlorométhane. Les phases organiques sont regroupées, lavées avec une solution de NaCl saturée, séchées sur sulfate de magnésium et évaporées à sec pour donner un solide beige. Ce solide brut est purifié par chromatographie sur colonne de silice (15g, éluant CH₂Cl₂/AcOEt 100/0 puis 97/3) pour conduire au composé substitué en N1 ( 189mg, 0.308mmol, 55.7%) sous forme de solide blanc et composé substitué en N2 (71mg, 0.116mmol, 20.9%, N2) sous forme de solide blanc.
Dérivé substitué en N1
¹H NMR (400 MHz, CDCl₃) : δ (ppm) = 1.29 (t ,3H, N-CH₂-CH₃), 1.50 (s, 9H, C(CH₃)₃), 1.53 (s, 9H, C(CH₃)₃), 3.31-3.47 (m, 4H, N-CH₂-CH-N et N-CH₂-CH₃), 3.80-3.90 (m, 1H, CH-CH₂-NH-C=NBoc), 4.00 (d, 1H, N-CH₂-CH-N), 4.30-4.40(m, 1H CH-CH2-NH-C=NBoc), 4.87 (AB, 2H, N-O-CH₂-Ph), 5.10-5.17(dd, 1H, CH-CH₂-NH-C=NBoc), 7.10 (broad, 1H , NH), 7.41- 7.46 (m, 6H, H pyrazole + Ph), 8.8 (s large, 1H, NH), 11. 40 (s, 1H, NH).
Dérivé substitué en N2
MS (ES(+) : m/z [M+H]⁺ = 613
¹H NMR (400 MHz, CDCl₃) : δ (ppm) = 1.27 (t, 3H, N-CH₂-CH₃), 1.54 (s, 9H, C(CH₃)₃), 1.55 (s, 9H, C(CH₃)₃), 3.06 (AB , 1H, N-CH₂-CH-N), 3.40 (AB, 1H, N-CH₂-CH-N), 3.43-3.50 (m, 2H, N-CH₂-CH₃), 3.85-3.95 (m, 2H, CH-CH₂-NH-C=NBoc), 3.98 (d, 1H, N-CH₂-CH-N), 4.73 (dd, 1H, CH-CH₂-NH-C=NBoc), 4.87-5.05 (AB, 2H, N-O-CH₂-Ph), 7.20 (broad, 1H, NH), 7.42- 7.45 (m, 5H O-CH₂-Ph), 8.07 (s, 1H, H de pyrazole), 8.9 (s large, 1H, NH), 11.50 (s, 1H, NH).

### Stade B

Sel de pyridinium du *trans*[[[1-éthylcarbamoyl-4,5,6,8-tétrahydro-6-oxo-5-(sulfooxy)-4,7-méthano-7*H*-pyrazolo[3,4-e][1,3] diazépin-8-yl]méthyl]-carbonimidoyl]-carbamate de bis-bis(1,1-diméthyléthyle)

En procédant comme indiqué au stade D de l'exemple 1, le dérivé N1 obtenu au stade précédent (150mg, 0.245mmol), le diméthylformamide (0.36mL), le dichlorométhane (1.1mL) et le palladium 10% sur charbon à 50% en eau (78.2mg, 0.037mmol) conduisent à l'intermédiaire débenzylé attendu.

L'intermédiaire débenzylé, la pyridine (0.74mL) et le complexe pyridine / sulfure de trioxyde (78mg, 0.245mmol) conduisent, après chromatographie sur colonne de silice (2g, éluant gradient 100/0, 95/5, puis 90/10) au dérivé attendu (78mg, 0.114mmol, 46.7%) sous forme de solide blanc.
¹H NMR (400 MHz, MeOH-d₄) : δ (ppm) = 1.29 (t, 3H, N-CH₂-CH₃), 1.54 (s, 9H, C(CH₃)₃), 1.61 (s, 9H, C(CH₃)₃), 3.41-3.47 (m, 3H;, N-CH₂-CH-N, et N-CH₂-CH₃) ; 3.60 ( AB , 1H, N-CH₂-CH-N) ; 3.71 (d, 1H, N-CH₂-CH-N), 3.82-3.83 (dd, 1H, CH-CH₂-NH-C=NBoc), 4.40-4.44(dd, 1H CH-CH2-NH-C=NBoc), 5.01-5.04(dd, 1H, CH-CH₂-NH-C=NBoc), 7.75 (s, 1H, H pyrazole).

### Stade C

### Sel de sodium du trans [[[1-éthylcarbamoyl-4,5,6,8-tétrahydro-6-oxo-5-(sulfooxy)-4,7-méthano-7H-pyrazolo[3,4-e][1,3] diazépin-8-yl]méthyl]-carbonimidoyl]-carbamate de bis-bis(1,1-diméthyléthyle)

En procédant comme indiqué au stade E de l'exemple 1, le sel obtenu au stade précédent (108mg , 0.158mmol) déposé en solution dans l'eau, la résine DOWEX 50WX8 (23g) et la soude 2N (120mL) conduisent au sel de sodium attendu (35mg, 0.056mmol, 35%)sous forme d'un lyophilisat blanc.
MS (ES(-)): m/z [M-H]⁻ = 601
¹H NMR (400 MHz, D₂O) : δ (ppm) = 1.12 (t, 3H, N-CH₂-CH₃), 1.37 (s, 9H, C(CH₃)₃), 1.43 (s, 9H, C(CH₃)₃), 3.30 (m, 2H, N-CH₂CH₃), 3.52 (m, 1H, N-CH₂-CH-N), 3.70-3.76 (m, 2H, N-CH₂-CH-N et CH-CH2-NH-C=NBoc), 4.27-4.33 (dd, 1H, CH-CH₂-NH-C=NBoc), 4.89 (s, 1H, N-CH₂-CH-N)), 4.96-5.10(m, 1H, CH-CH₂-NH-C=NBoc), 7.70 (s, 1H, H pyrazole).

### Stade D

### Sel de sodium et de trifluoroacétate de la trans 8-(guanidino-méthyl)-1-éthylcarbamoyl-4,8-dihydro-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (35mg, 0.055mmol), le dichlorométhane (4mL), l'acide trifluoroacétique (2mL) conduisent au produit attendu (22mg, 33mmol, 61%) sous forme de solide brun.
MS (ES(-)): m/z [M-H]⁻ = 401
¹H NMR (400 MHz, DMSO-d_{6 +} 1 goutte D₂O) : δ (ppm) = 1.1-1.2 (m , 3H, N-CH₂-CH₃), 3.2-3.4 (m, 2H, N-CH₂-CH₃), 3.5-4.00 (m, 4H, CH-CH2-NH-C=N et N-CH₂-CH-N), 4.63-4.70(m, 1H, N-CH₂-CH-N), 4.8(s, 1H, CH-CH₂-NH-C=N), 7.75 / 8.05 (s, H Pyrazole).

### Exemple 16 : Sel de sodium et de trifluoroacétate de la trans 8-(guanidino-méthyl)-2-éthylcarbamoyl-4,8-dihydro-5-(sulfooxy)-4,7-méthano-7H-pyrazolo[3,4-e][1,3]diazépin-6(5H)-one

### Stade A

Sel de pyridinium du *trans* [[[2-éthylcarbamoyl-4,5,6,8-tétrahydro-6-oxo-5-(sulfooxy)-4,7-méthano-7*H*-pyrazolo[3,4-e][1,3] diazépin-8-yl]méthyl]-carbonimidoyl]-carbamate de bis-bis(1,1-diméthyléthyle)

En procédant comme indiqué au stade D de l'exemple 1, le dérivé N2 obtenu au stade A de l'exemple 15 (80mg, 0.130mmol), le diméthylformamide (0.19mL), le dichlorométhane (0.76mL) et le palladium 10% sur charbon à 50% en eau (42mg, 0.019mmol) conduisent à l'intermédiaire débenzylé attendu. (MS (ES(+) : m/z [M+H]⁺ = 523)

L'intermédiaire débenzylé, la pyridine (0.39mL) et le complexe pyridine / sulfure de trioxyde (41.4mg, 0.260mmol) conduisent, après chromatographie sur colonne de silice (2g, éluant CH₂Cl₂/MeOH 100/0, 95/5, 90/10 puis 85/15) au produit attendu (51mg, 0.074mmol, 57%) sous forme d'un solide beige.
MS (ES(+) : m/z [M+H]⁺ = 603 , m/z [M+H-(Boc)]⁺ = 503
¹H NMR (400 MHz, MeOH-d₄) : δ (ppm) = 1.26 (t , 3H, N-CH₂-CH₃), 1.54 (s, 9H, C(CH₃)₃), 1.61 (s, 9H, C(CH₃)₃), 3.43-3.47 (m, 2H, N-CH₂-CH₃), 3.52 (AB , 1H, N-CH₂-CH-N), 3.66 (AB, 1H, N-CH₂-CH-N), 3.82 (dd, 1H, CH-CH₂-NH-C=NBoc), 4.09 (dd, 1H, CH-CH₂-NH-C=NBoc), 4.77 (dd, 1H, N-CH₂-CH-N), 5.00 (dd, 1H, CH-CH₂-NH-C=NBoc), 8.31(s, 1 H , H de pyrazole),

### Stade B

### Sel de sodium du trans [[[2-éthylcarbamoyl-4,5,6,8-tétrahydro-6-oxo-5-(sulfooxy)-4,7-méthano-7H-pyrazolo[3,4-e][1,3] diazépin-8-yl]méthyl]-carbonimidoyl]-carbamate de bis-bis(1,1-diméthyléthyle)

En procédant comme indiqué au stade E de l'exemple 1, le sel obtenu au stade précédent (49mg, 0.072mmol) déposé en solution dans l'eau, la résine DOWEX 50WX8 (8g) et la soude 2N (50mL) conduisent au sel de sodium attendu (18mg, 0.029mmol, 40%) sous forme d'un lyophilisat blanc.
MS(ES(-)): m/z [M-H]⁻ = 601,) m/z [M-H-(Boc)]⁻ = 501
¹H NMR (400 MHz, D₂O) : δ (ppm) = 1.14 (t, 3H, N-CH₂-CH₃), 1.34 (s, 9H, C(CH₃)₃), 1.44 (s, 9H, C(CH₃)₃), 3.29-3.34 (m, 2H, N-CH₂-CH₃), 3.52-3.62 (m, 2H, CH-CH2-NH-C=NBoc), 3.72-3.77 (m, 1H, N-CH₂-CH-N), 3.87-3.92 (m, 1H, N-CH₂-CH-N), 4.75 (s, 1H, N-CH₂-CH-N), 5.00(m, 1H, CH-CH₂-NH-C=NBoc), 8.22 (s, 1H, H pyrazole).

### Stade C

### Sel de sodium et de trifluoroacétate de la trans 8-(guanidino-méthyl)-2-éthylcarbamoyl-4,8-dihydro-5-(sulfooxy)-4,7-méthano-7H-pyrazolo[3,4-e][1,3] diazépin-6(5H)-one

En procédant comme indiqué au stade F de l'exemple 1, le sel de sodium obtenu au stade précédent (18mg, 0.028mmol), le dichlorométhane (3mL), l'acide trifluoroacétique (1.5mL) conduisent au produit attendu. La suspension obtenue en reprenant le produit dans l'eau est filtrée pour éliminer l'insoluble. Le filtrat est congelé et lyophilisé pour donner le produit attendu (12mg, 0.018mmol, 64.5%) sous forme d'un solide légèrement brun.
MS (ES(-)): m/z [M-H]⁻ = 401
¹H NMR (400 MHz, DMSO-d₆₊ 1goutte D₂O) : δ (ppm) = 1.08-1.12 (m, 3H, N-CH₂-CH₃), 3.24-3.29 (m, 2H, N-CH₂-CH₃) ; 3.40 (broad, 2H, CH-CH2-NH-C=N) ; 3.63 (s, large, pic H₂O du DMSO-d₆ et 2H du N-CH₂-CH-N) ; 3.51-3.55(t, 1H, N-CH₂-CH-N),), 4.84 (s, 1H, CH-CH₂-NH-C=N), 8.23 (s, 1H, H pyrazole).

### Exemple 17 : trans 8-(guanidino-méthyl)-2-carbamoyl-4,8-dihydro-5-(sulfooxy)-4,7-méthano-7H-pyrazolo[3,4-e][1,3]diazépin-6(5H)-one

### Stade A

### trans [[[2-carbamoyl-4,5,6,8-tétrahydro-6-oxo-5-(phénylméthoxy)-4,7-méthano-7H-pyrazolo[3,4-e] [1,3] diazépin-8-yl]méthyl]-carbonimidoyl]-carbamate de bis-bis(1,1-diméthyléthyle)

Le produit obtenu au stade A de l'exemple 13 (0.114g, 0.21mmol) est mis en solution dans un mélange acétonitrile/ tétrahydrofurane anhydres 40/60(3.5mL), sous azote. La solution est refroidie par un bain eau-glace. Puis l'isocyanate de triméthylsilane (0.030mL, 0.21mmol) est ajouté goutte à goutte. Après 50mn d'agitation, le bain eau-glace est enlevé. Le mélange réactionnel est agité à température ambiante. Après une nuit, l'analyse par HPLC indiquait la présence d'encore 82% de produit de départ. Le mélange réactionnel est alors refroidi à 0°C et de l'isocyanate de triméthylsilane (0.030mL, 0.21mmol) est ajouté. Une nouvelle addition d'isocyanate de triméthylsilane (0.030mL, 0.21mmol) est répétée après 30h, et l'agitation est poursuivie pendant une nuit. Le mélange réactionnel est traité par ajout d'eau (1mL) et de méthanol (1mL). Le mélange est agité pendant 1h puis évaporé à sec. Le solide obtenu est repris par 5mL d'eau suivi de 30mn d'agitation, un précipité blanc est observé. Celui-ci est filtré et séché sous vide pour donner 125mg de produit brut.

La réaction est mise en oeuvre une deuxième fois sur 150mg (0.277mmol) de substrat en utilisant 0.279mL (1.94mmol) d'isocyanate de triméthylsilane (ajouté en 4 fois sur 6 jours), pour donner 160mg de produit brut.

Les deux lots bruts sont regroupés et purifiés par chromatographie sur colonne de silice (10g, éluant CH₂Cl₂/AcOEt 100/0 , 95/05, 91/09 puis 85/15). Ceci permet d'isoler le composé attendu substitué en N2 (72mg, 0.123mmol, 25%) sous forme de solide blanc.
¹H NMR (400 MHz, CDCl₃) : δ (ppm) =1.50 (s, 9H, C(CH₃)₃), 1.53 (s, 9H, C(CH₃)₃), 3.00 (AB , 1H, N-CH₂-CH-N), 3.40 (AB, 1H, N-CH₂-CH-N), 3.78-3.85 (m, 1H, CH-CH₂-NH-C=NBoc), 3.99 (d, 1H, N-CH₂-CH-N), 4.00-408 (m, 1H, CH-CH₂-NH-C=NBoc), 4.74 (dd, 1H, CH-CH₂-NH-C=NBoc), 4.87-5.05 (AB, 2H, N-O-CH₂-Ph), 5.25 (broad, 1H, NH), 7.30 (s , 1H, NH), 7.42- 7.45 (m, 5H O-CH₂-Ph), 8.07 (s, 1H ,H de pyrazole), 9.00 (s large, 1H, NH), 11. 60 (s, 1H, NH).

### Stade B

Sel de pyridinium du *trans*-[[[2-carbamoyl-4,5,6,8-tétrahydro-6-oxo-5-(sulfooxy)-4,7-méthano-7*H*-pyrazolo[3,4-e][1,3] diazépin-8-yl]méthyl]-carbonimidoyl]-carbamate de bis-bis(1,1-diméthyléthyle)

En procédant comme indiqué au stade D de l'exemple 1, le dérivé obtenu au stade précédent (86mg, 0.147mmol), le diméthylformamide (0.21mL), le dichlorométhane (0.64mL) et le palladium 10% sur charbon à 50% en eau (47mg, 0.022mmol) conduisent à l'intermédiaire débenzylé attendu.

L'intermédiaire débenzylé, la pyridine anhydre(0.4mL) et le complexe pyridine / sulfure de trioxyde (47mg, 0.294mmol, renouvelé au bout de une nuit) conduisent, après chromatographie sur colonne de silice (2g, éluant 100/0, 95/5, puis 90/10) au produit attendu (57mg, 0.087mmol, 59%) sous forme de solide blanc.
MS (ES(-)): m/z [M-H]⁻ = 573,) m/z [M-H-(Boc)]⁻ = 473
¹H NMR (400 MHz, MeOH-d₄) : δ (ppm) = 1.50 (s, 9H, C(CH₃)₃), 1.62 (s, 9H, C(CH₃)₃); 3.49 (AB, 1H, N-CH₂-CH-N) ; 3.69 (AB, 1H, N-CH₂-CH-N) ; 3.98-4.02 (m, 2H, CH-CH₂-NH-C=NBoc) ; 4.79 (dd, 1H, N-CH₂-CH-N), 5.02 (d, 1H, CH-CH₂-NH-C=NBoc) : 8.34 (s, 1H, H de pyrazole).

### Stade C

### Sel de sodium du trans [[[2-carbamoyl-4,5,6,8-tétrahydro-6-oxo-5-(sulfooxy)-4,7-méthano-7H-pyrazolo[3,4-e][1,3] diazépin-8-yl]méthyl]-carbonimidoyl]-carbamate de bis-bis(1,1-diméthyléthyle)

En procédant comme indiqué au stade E de l'exemple 1, le sel obtenu au stade précédent (57mg, 0.087mmol) déposé en solution dans un minimum d'eau, la résine DOWEX 50WX8 (10g) et la soude 2N (50mL) conduisent au sel de sodium attendu (24mg, 0.04mmol, 46%) sous forme d'un lyophilisat blanc.
MS (ES(-)): m/z [M-H]⁻ = 573
¹H NMR (400 MHz, D₂O) : δ (ppm) = 1.36 (s, 9H, C(CH₃)₃), 1.43 (s, 9H, C(CH₃)₃), 3.56 (AB , 2H, N-CH₂-CH-N), 3.85 (d, 2H, CH-CH₂-NH-C=NBoc), 4.75 (broad, 1H, CH-CH₂-NH-C=NBoc), 4.95 (s, 1H, N-CH₂-CH-N), 8.25 (s, 1H, H de pyrazole).

### Stade D

### trans [[8-(guanidino-méthyl)-2-carbamoyl-4,8-dihydro-5-(sulfooxy)-4,7-méthano-7H-pyrazolo[3,4-e] [1,3] diazépin-6(5H)-one

En procédant comme indiqué au stade F de l'exemple 1, le sel de sodium obtenu au stade précédent (24mg, 0.04mmol), le dichlorométhane (3mL), l'acide trifluoroacétique (3mL) conduisent au produit attendu. Après évaporation sous vide, le rpoduit est repris dans l'eau. La suspension obtenue est filtrée. Le solide est récupéré et séché sous vide pendant une nuit pour donner le composé attendu (4.9mg, 0.013mmol, 32.5%) sous forme de solide beige.
MS (ES(-)) : m/z [M-H]⁻ = 473
¹H NMR (400 MHz, DMSO-d₆₊ 1 goutte D₂O) : δ (ppm) = 3.40 (m, 2H, N-CH₂-CH-N), 3.60 (m, 2H, CH-CH₂-NH-C=N), 4.52 (broad, 1H, CH-CH₂-NH-C=N), 4.84 (s, 1H, N-CH₂-CH-N), 8.23 (s, 1H, H de pyrazole).

### Exemple 18 : Sel de sodium et de trifluoroacétate de la trans 8-(amino-méthyl)-4,8-dihydro-1-méthyl-6-oxo-5-(carboxy-difluoro-méthoxy)-4,7-méthano-7H-pyrazolo[3,4-e][1,3]diazépin-6(5H)-one

### Stade A

### trans [[4,8-dihydro-1-methyl-6-oxo-5-(phenylmethoxy)-4,7-methano-7H-pyrazolo[3,4-e] [1,3] diazépin-8-yl]méthyl]-carbamate de 1,1-diméthyléthyle

Une solution molaire de triméthylphosphine (3.4mL, 3.4mmol) est ajoutée goutte à goutte à une solution de l'azoture obtenu au stade E de l'exemple 14 (1.15g, 3.39mmol) dans un mélange toluène (5mL) et tétrahydrofurane (5mL) à température ambiante sous azote et sous agitation. Après 3h d'agitation, une solution de BOC-ON (0.92g, 3.6mmol) dans le tétrahydrofurane (10mL) est ajoutée goutte à goutte au milieu réactionnel refroidi à 0°C. L'agitation est poursuivie pendant 3h à température ambiante. Le milieu réactionnel est traité par une solution aqueuse à 10% de NaHCO₃ (50mL). La phase aqueuse est extraite à l'acétate d'éthyle (50mL). La phase organique est séchée, puis concentrée sous pression réduite pour donner une huile (2.2g). Le produit brut est chromatographié sur colonne de silice (éluant cyclohexane/ acétate d'éthyle 5/5), pour donner le produit attendu (0.62g, 1.49mmol, 70%). MS (ES(+)) : m/z [M⁺] = 414
¹H NMR (400MHz, CDCl3): δ (ppm) = 1.39 (s, 9H, C(CH₃)₃), 3.05 (dd, 1H, N-CH₂-CH-N), 3.19 (dd, 1H, CH-CH₂-NHBoc), 3.27 (dd, 1H, N-CH₂-CH-N), 3.72 (s, 3H, CH₃), 3.78(m, 1H, CH-CH₂-NHBoc), 3.88 (d, 1H, N-CH₂-CH-N), 4.48 (dd, 1H, CHCH₂NHBoc), 4.79 (d, 1H, N-O-CH₂-Ph), 4.92 (d, 1H, N-O-CH₂-Ph), 5.18 (m, 1H, NH), 7.35 (s, 1H, H pyrazole), 7.37-7.48 (massif, 5H, Ph)

### Stade B

### trans [[4,8-dihydro-1-méthyl-6-oxo-5-(éthoxycarbonyl-difluoro-méthoxy)-4,7-méthano-7H-pyrazolo[3,4-e] [1,3] diazépin-8-yl]méthyl]-carbamate de 1,1-diméthyléthyle

Du palladium 10% sur charbon (70mg) est ajouté à une solution de l'amine obtenue au stade A de l'exemple 10 (300mg, 0.72mmol) dans le méthanol (5mL). Le milieu réactionnel est hydrogéné pendant 3h. Le méthanol est ensuite évaporé sous pression réduite pour donner l'intermédiaire débenzylé.
MS (ES(+)) : m/z [M⁺] = 324

L'intermédiaire débenzylé (234mg, 0.72mmol) est repris, sous azote dans le diméthylformamide anhydre (1.6mL) en présence de carbonate de potassium (0.25g, 1.81mmol) et de bromodifluoroacétate d'éthyle (0.373mL, 2.89mmol). La réaction est maintenue sous agitation à température ambiante pendant une nuit. Le mélange réactionnel est ensuite filtré et rincé par l'acétate d'éthyle. Le filtrat est lavé trois fois à l'eau, la phase organique est séchée sur du sulfate de magnésium puis concentrée sous pression réduite. Le brut réactionnel est chromatographié sur colonne de silice (éluant gradient CH₂Cl₂/méthanol 100/0 à 95/5) pour conduire au dérivé attendu (185mg, 0.42mmol, 57%) sous la forme d'une huile.
MS (ES(+)) : m/z [M⁺] = 446
¹H NMR (400 MHz, CDCl₃) : δ (ppm) = 1.37 (t, 3H, CH₂-CH₃), 1.60 (s, 9H, C(CH₃)₃), 3.24 (m, 1H, CH-CH₂-NHBoc), 3.33 (m, 1H, N-CH₂-CH-N), 3.53 (dd, 1H, N-CH₂-CH-N), 3.80-3.95 (m, 4H, CH₃ et CH-CH₂-NHBoc), 4.37 (m, 2H, CH₂-CH₃), 4.60-4.72 (m, 2H, N-CH₂-CH-N et CH-CH₂-NHBoc), 5.16 (broad, 1H, NH), 7.48 (s, 1H, H pyrazole)

### Stade C

### trans [[4,8-dihydro-1-méthyl-6-oxo-5-(carboxy-difluoro-methoxy)-4,7-méthano-7H-pyrazolo[3,4-e] [1,3] diazépin-8-yl]méthyl]-carbamate de 1,1-diméthyléthyle

De l'hydroxyde de lithium (0.017g, 0.41mmol) est ajouté en deux fois à une solution de l'ester obtenu au stade B de l'exemple 10 (0.181g, 0.41mmol) dans un mélange tétrahydrofurane/eau (7.38/2.48mL) à 0°C. L'agitation est poursuivie à 0°C pendant 1h30. Le milieu réactionnel est traité par une solution aqueuse d'acide tartrique à 10% et extrait 2 fois par l'acétate d'éthyle. La phase organique est séchée sur du sulfate de magnésium, puis concentrée sous vide pour conduire à l'acide attendu (110mg, 0.32mmol, 79%) sous la forme d'un amorphe.
MS (ES(+)) : m/z [M⁺] = 418
¹H NMR (400 MHz, MeOH-*d₄*): δ (ppm) = 1.53 (s, 9H, C(CH₃)₃), 3.42-3.58 (m, 3H, CH-CH₂-NHBoc et N-CH₂-CH-N), 3.76 (dd, 1H, N-CH₂-CH-N), 3.90 (s, 3H, CH₃), 4.70-4.80 (m, 2H, N-CH₂-CH-N et CH-CH₂-NHBoc), 7.54 (s, 1H, H pyrazole).

### Stade D

Sel de sodium de la *trans* [[4,8-dihydro-1-méthyl-6-oxo-5-(carboxy-difluoro-méthoxy)-4,7-méthano-7*H*-pyrazolo[3,4-e][1,3] diazépin-8-yl]méthyl]-carbamate de 1,1-diméthyléthyle De la triéthylamine (0.037mL, 0.26mmol) est ajoutée à 0°C à une solution de l'acide obtenu au stade C de l'exemple 10 (0.110mg, 0.26mmol) dans le tétrahydrofurane (3mL). L'agitation est poursuivie 1h à 0°C puis le mélange réactionnel est concentré sous vide pour conduire au sel de triéthylamine correspondant (0.135g, 0.26mmol). Une suspension de 16g de résine DOWEX 50WX8 dans une solution de soude 2N (80mL) est agitée pendant 1h, puis transvasée sur une colonne à chromatographier. La colonne est éluée à l'eau déminéralisée jusqu'à pH neutre, puis conditionnée avec un mélange eau/THF 90/10. Le sel de triéthylamine précédemment obtenu (0.135g, 0.26mmol) est dissout dans un minimum d'eau, déposé sur la colonne, puis élué avec un mélange eau/THF 90/10. Les fractions contenant le substrat sont réunies, congelées puis lyophilisées pour conduire au sel de sodium attendu (0.088g, 0.20mmol, 64%) sous la forme d'un amorphe.
MS (ES(-)) : m/z [M⁻] = 415
¹H NMR (400 MHz, MeOH-*d₄*): δ (ppm) = 1.53 (s, 9H, C(CH₃)₃), 3.44-3.58 (m, 3H, CH-CH₂-NHBoc et N-CH₂-CH-N), 3.74 (dd, 1H, N-CH₂-CH-N), 3.90 (s, 3H, CH₃), 4.69-4.83 (m, 2H, N-CH₂-CH-N et CH-CH₂-NHBoc), 7.54 (s, 1H, H pyrazole).

### Stade E

Sel de sodium et de trifluoroacétate de la *trans* [[8-(amino-méthyl)-4,8-dihydro-1-méthyl-6-oxo-5-(carboxy-difluoro-méthoxy)-4,7-méthano-7*H*-pyrazolo[3,4-e][1,3] diazépin-6(5*H*)-one Une solution d'acide trifluoroacétique (4.18mL) dans le dichlorométhane (4.18mL) est coulée goutte à goutte sur une solution du sel de sodium obtenu au stade D de l'exemple 10 (66mg, 0.15mmol) dans le dichlorométhane (2.11mL) sous azote et refroidie à 0°C. La réaction est maintenue sous agitation pendant 3h à 0°C. Le mélange est évaporé à sec, repris dans l'acétone, trituré et mis au réfrigérateur pour la nuit. Le précipité formé est filtré, rincé à l'acétone et séché sous vide pour conduire au sel de sodium et de trifluoroacétate attendu (40mg, 0.088mmol, 59%) sous la forme d'une poudre beige clair.
MS (ES(+)) : m/z [M⁺] = 318
¹H NMR (400 MHz, DMSO-*d*₆) : δ (ppm) = 3.27-3.60 (m, 4H, CH-CH₂-NH;⁺ et N-CH₂-CH-N), 3.75 (s, 3H, CH₃), 4.71 (d, 1H, N-CH₂-CH-N), 4.82 (t, 1H, CH-CH₂-NH₃⁺), 7.44 (s, 1H, H pyrazole), 8.17 (broad, 3H, NH₃⁺).

### Exemple 19 : Sel de sodium et de trifluoroacétate de la trans 8-(aminométhyl)-2-(amino-carbamoyle)-4,8-dihydro-5-(sulfooxy)-4,7-méthano-7H-pyrazolo[3,4-e][1,3]diazépin-6(5H)-one

### Stade A

### trans [2-(2-tert-butoxycarbonylamino-carbamoyle-4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyléthyle

En procédant comme indiqué au stade C de l'exemple 1, le dérivé obtenu au stade B de l'exemple 1 (1g, 2.5mmol)), le dichlorométhane (131 mL), la triéthylamine (698 µL), le diphosgène (453 µL) et la N-Boc-hydrazine (1.158g) conduisent, après chromatographie sur colonne de silice (éluant gradient 100/0 à 95/5), au dérivé attendu (654 mg, 1.17 mmol mmol, 47%).
MS (ES(+)) : m/z [M+H]⁺ = 558
¹H RMN (400MHz, DMSO-*d*₆) : δ(ppm) = 1.40 (s, 9H, (C(CH₃)₃)), 1.42 (s, 9H, (C(CH₃)₃)), 3.23-3.41 (m, 4H, N-CH₂-CH-N et NCH-CH₂-NHBOC), 4.39 (m, N-CH-CH₂-NHBOC), 4.62 (s, 1H, N-CH₂-CH-N), 4.91 (m, 2H, CH₂Ph), 7.13 (m, 1H, NH), 7.35-7.43 (m, 5H, Ph), 8.30 (s, 1H, pyrazole), 9.10 (s, 1H, NH), 10.22 (s, 1H, NH).

### Stade B

### Sel de sodium du trans [2-(2-tert-butoxycarbonylamino-carbamoyle -4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, le dérivé obtenu au stade précédent (654 mg, 1.17 mmol), le diméthylformamide (3.3 mL), le dichlorométhane (10 mL) et le palladium 10% sur charbon à 50% en eau (262 mg) conduisent à l'intermédiaire débenzylé attendu.

L'intermédiaire débenzylé, la pyridine (4.5 mL) et le complexe pyridine / sulfure de trioxyde (373 mg) conduisent, après chromatographie sur colonne de silice (éluant gradient 80/20) au dérivé attendu (43 mg) sous forme d'un solide blanc.

Une suspension de 75g de résine DOWEX 50WX8 dans une solution de soude 2N (375mL) est agitée pendant 1h, puis versée sur une colonne à chromatographie. La colonne est conditionnée à l'eau déminéralisée jusqu'à pH neutre. Le dérivé obtenu (431mg) est dissout dans H₂O, déposé sur la colonne, puis élué avec H₂O. Les fractions contenant le substrat sont réunies, congelées et lyophilisées pour conduire au sel de sodium attendu (362mg, 0.63mmol, 54%) sous forme d'une poudre blanche.
MS (ES (-)) : m/z [M-H]⁻ = 546
¹H NMR (400MHz, DMSO-*d₆*): δ (ppm) = 1.42 (s, 18H, (C(CH₃)₃), 3.16-3.60 (m, 4H, N-CH₂-CH-N et N-CH-CH₂-NHBOC), 4.38 (t, 1H, N-CH-CH₂-NHBOC), 4.79 (s, 1H, N-CH₂-CH-N), 7.14 (m, 1H, NH), 8.23 (s, 1H, pyrazole), 9.17 (s, 1H, NH), 10.18 (s, 1H, NH).

### Stade C

Sel de sodium et de trifluoroacétate de la trans 8-(aminométhyl)-2-(amino-carbamoyle)-4,8-dihydro-5-(sulfooxy)-4,7-méthano-7H-pyrazolo[3,4-e][1,3]diazépin-6(5H)-one

Le composé obtenu au stade B (362mg, 0.635mmol) est placé en suspension dans du dichlorométhane anhydre (0.75mL), sous azote. Un mélange acide trifluoroacétique/dichlorométhane 1/1 (0.944mL) est ensuite ajouté goutte à goutte puis la réaction est poursuivie à température ambiante pendant trois heures. Après évaporation à sec, le produit est ensuite repris dans l'eau, gelé puis lyophilisé pour donner le produit attendu sous forme de poudre blanche (363mg, 0.607mmol, 96%).
MS (ES (-)) : m/z [M-H]⁻ = 346
¹H NMR (400MHz, DMSO-*d₆*): δ (ppm) = 3.07-4.19 (m, 4H, N-CH₂-CH-N et CH-CH₂-NH₂), 4.68 (dd, 1H, CH-CH₂-NH₃⁺), 4.70 (m, 1H, N-CH₂-CH-N), 8.15 (broad s, 3H, NH), 8.30 (s, 1H, H pyrazole).

### Composition pharmaceutique

On a préparé une composition pour injection renfermant:
- Composé de l'exemple 1: 500 mg
- Excipient aqueux stérile: q.s.p. 5 cm³

### ETUDE PHARMACOLOGIQUE DES COMPOSES DE L'INVENTION

### Activité in vitro, méthode des dilutions en milieu liquide :

On prépare une série de tubes dans lesquels on répartit la même quantité de milieu nutritif stérile, on distribue dans chaque tube des quantités croissantes du produit à étudier puis chaque tube est ensemencé avec une souche bactérienne. Après incubation de 24 heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits des exemples 1 à 19 en comparaison avec les produits des exemples 11 de la demande WO 04/052891 et 18 de la demande WO 02/100860. Les produits de la présente demande se révèlent très actifs sur Pseudomonas aeruginosa, ce qui n'est absolument pas le cas des produits de comparaison.

La différence d'activité sur Pseudomonas aeruginosa entre les produits de l'invention et les produits les plus proches de l'art antérieur se situe selon les produits à un niveau pouvant aller jusqu'à plus de 1000 fois plus actifs.

### Activité sur Pseudomonas aeruginosa (1771 Souche type sauvage)

| Molécules | MIC (µg/mL) 24h (P, aerug, 1771) |
|---|---|
| Ex1 | 0.12 |
| Ex2 | 0.5 |
| Ex3 | 1 |
| Ex4 | 8 |
| Ex5 | 0.5 |
| Ex6 | 4 |
| Ex7 | 1 |
| Ex9 | 0.5 |
| Ex10 | 0.5 |
| Ex11 | 0.25 |
| Ex12 | 0.5 |
| Ex13 | 1 |
| Ex14 | 1 |
| Ex15 | >32 |
| Ex16 | 16 |
| Ex17 | 1 |
| Ex18 | 0.5 |
| Ex19 | 0.5 |
| Ex 11 Demande de brevet WO 04/052891 | >160 |
| Ex 18 Demande de brevet WO 02/100860 | >160 |

## Revendications

1. Les composés de formule (I), sous leurs formes isomères ou diastéroisomères possibles, ou de mélanges: 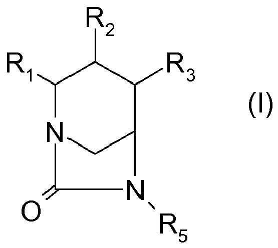 dans laquelle :
R₁ représente un radical -(CH₂)ₘ-NH₂,
-(CH₂)ₘ-NH(C₁-C₆)alc, -(CH₂)ₘ-N(C₁-C₆)alc₂, -(CH₂)ₘ-NH-C(NH)NH₂ ou -(CH₂)ₘ-NH-CH=NH, dans lequel m est égal à 1 ou 2;
R₂ et R₃ forment ensemble un hétérocycle azoté à caractère aromatique à 5 sommets renfermant 1, 2 ou 3 atomes d'azote, substitué sur un atome d'azote par R₄ ;
R₄ représente un atome d'hydrogène, un radical (C₁-C₆)alc ou une chaîne de formule :
-(A)ₙ-(NH)ₒ-(CH₂)ₚ-(CHR')_{q}R" ;
A représente un groupe C=O, C=NH ou SO₂;
R' représente un atome d'hydrogène ou un groupe carboxy;
R" représente un atome d'hydrogène ou un groupe NH₂, NH(C₁-C₆)alc, N(C₁-C₆)alc₂, CONH₂, CONH(C₁-C₆)alc, CON(C₁-C₆)alc₂, ou un hétérocycle saturé à 5 ou 6 sommets renfermant 1 ou 2 atomes d'azote et, le cas échéant, un autre hétéroatome choisi parmi l'oxygène et le soufre, fixé à la chaîne par un atome d'azote ou par un atome de carbone et éventuellement substitué par un radical (C₁-C₆)alc ;
n, o et q représentent 0 ou 1 et p représente un entier de 0 à 4 ;
R₅ représente un groupe OSO₃H ou OCHFCO₂H ou OCF₂CO₂H;
étant entendu que :
- R₁ est différent de -(CH₂)ₘ-NH₂, -(CH₂)ₘ-NH(C₁-C₆)alc ou -(CH₂)ₘ-N(C₁-C₆)alc₂
lorsque R₄ est hydrogène, -(C₁-C₆)alc, -(C=O)ₙ-(CH₂)₍₀₋₅₎-NH₂, -(C=O)ₙ-(CH₂)₍₀₋₅₎-NH(C₁-C₆)alc ou -(C=O)ₙ-(CH₂)₍₀₋₅₎-N(C₁-C₆)alc₂ et R₅ est un groupe OSO₃H,
ou lorsque R₄ a l'ensemble des valeurs de R" ci-dessus à l'exception d'hétérocycle tel que défini plus haut,
- et n, o, p et q ne peuvent être tous égaux à 0 sauf lorsque R" est hydrogène ou un groupe CONH₂, CONH(C₁-C₆)alc, CON(C₁-C₆)alc₂ ou un hétérocycle ;
sous forme libre et sous forme de zwitterions et de sels avec les bases et les acides minéraux ou organiques pharmaceutiquement acceptables.

2. Les composés de formule générale (I) selon la revendication 1, dans lesquels R₂ et R₃ forment ensemble un hétérocycle pyrazolyle.

3. Les composés de formule générale (I) selon la revendication 1 ou 2, **caractérisés en ce que** R₁ représente un radical -(CH₂)ₘ-NH₂, m étant égal à 1.

4. Les composés de formule générale (I) selon la revendication 1 ou 2, **caractérisés en ce que** R₁ représente un radical -(CH₂)ₘ-NH-C(NH)NH₂, m étant égal à 1.

5. Les composés de formule générale (I) selon l'une quelconque des revendications 1 à 4, **caractérisés en ce que** R₄ représente une chaîne de formule -(A)ₙ-(NH)ₒ-(CH₂)ₚ-(CHR')q R" telle que définie à la revendication 1.

6. Les composés de formule générale (I) selon l'une quelconque des revendications 1 à 5, **caractérisés en ce que** R₄ représente une chaîne de formule -C(O)-NH-(CH₂)ₚ-(CHR')_{q} R" dans laquelle R', R", p et q sont tels que définis à la revendication 1.

7. Les composés de formule générale (I) selon l'une quelconque des revendications 1 à 4, **caractérisés en ce que** R₄ représente un atome d'hydrogène ou un radical (C₁-C₆)alc et R₁ représente un radical -(CH₂)ₘ-NH-C(NH)NH₂ ou -(CH₂)ₘ-NH-CH=NH, dans lequel m est égal à 1.

8. L'un quelconque des composés de formule générale (I) selon la revendication 1, dont les noms suivent:
- la trans 8-(aminométhyl)-2-(2-amino-éthyl-carbamoyl)-4,8-dihydro-5-(sulfooxy)-4,7-méthano-7H-pyrazolo[3,4-e] [1,3]diazépin-6(5H)-one,
- la trans 8-(aminométhyl)-2-(4-pipérazine-1-carbonyl)-4,8-dihydro-5-(sulfooxy)-4,7-méthano-7H-pyrazolo[3,4-e][1,3] diazépin-6(5H)-one,
- la trans 8-(aminométhyl)-4,8-dihydro-2-(2-diméthylamino-éthyl-carbamoyl)-5-(sulfooxy)-4,7-méthano-7H-pyrazolo[3,4-e] [1,3] diazépin-6(5H)-one,
- la trans 8-(aminométhyl)-2-(3-amino-propyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-2-(carbamoylméthyl-carbamoyl)-4,8-dihydro-5-(sulfooxy)-4,7-méthano-7H-pyrazolo[3,4-e] [1,3] diazépin-6(5H)-one,
- la trans 8-(aminométhyl)-1-(carbanimidoyl)-4,8-dihydro-5-(sulfooxy)-4,7-méthano-7H-pyrazolo[3,4-e] [1,3] diazépin-6(5H)-one,
- la trans 8-(aminométhyl)-2-(carbanimidoyl)-4,8-dihydro-5-(sulfooxy)-4,7-méthano-7H-pyrazolo[3,4-e] [1,3] diazépin-6(5H)-one,
- la trans 8-(amino-méthyl)-4,8-dihydro-2-(pipéridine-4-carbonyl)-5-(sulfooxy)-4,7-méthano-7H-pyrazolo[3,4-e] [1,3] diazépin-6(5H)-one,
- la trans 8-(amino-méthyl)-2-(3-amino-3-carboxy-propyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-5-(sulfooxy)-4,7-méthano-7H-pyrazolo[3,4-e] [1,3] diazépin-6(5H)-one,
- la trans 8-(guanidino-méthyl)-4,8-dihydro-1-méthyl-5-(sulfooxy)-4,7-méthano-7H-pyrazolo[3,4-e] [1,3] diazépin-6(5H)-one,
- la trans 8-(guanidino-méthyl)-2-carbamoyl-4,8-dihydro--(sulfooxy)-4,7-méthano-7H-pyrazolo[3,4-e] [1,3] diazépin-6(5H)-one,
- la trans 8-(amino-méthyl)-4,8-dihydro-1-méthyl-5-(carboxy-difluoro-méthoxy)-4,7-méthano-7H-pyrazolo[3,4-e][1,3] diazépin-6(5H)-one,
- la trans 8-(amino-méthyl)-2-amino-carbamoyle-4,8-dihydro-5-(sulfooxy)-4,7-méthano-7H-pyrazolo[3,4-e] [1,3] diazépin-6(5H)-one,
sous forme libre, de zwittterions et de sels avec les bases et les acides minéraux ou organiques pharmarceutiquement acceptables et sous ses formes isomères ou diastéroisomères possibles, ou de mélanges.

9. Procédé de préparation de composés de formule (I), telle que définie à la revendication 1, **caractérisé en ce que** l'on traite un composé de formule (II) : 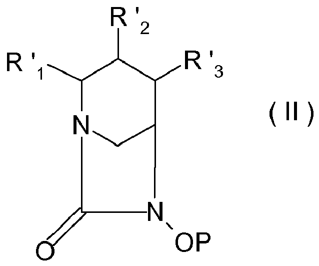 das laquelle R'₁ représente un radical R₁ dans lequel, le cas échéant, la ou les fonctions amino présentes sont protégées, R'₂ et R'₃ forment ensemble un hétérocycle azoté à caractère aromatique à 5 sommets renfermant 1, 2 ou 3 atomes d'azote et P représente un groupement protecteur du radical hydroxy, en présence d'une base, par le diphosgène, puis par une amine de formule (III) :
H-NH-(CH₂)ₚ-(CHR'ₐ)_{q}R'" (III)
dans laquelle R'ₐ et R"' représentent repectivement R' et R" dans lesquels, le cas échéant, les fonctions réactives carboxy et amino sont protégées, et p et q sont tels que définis à la revendication 1, pour obtenir un composé de formule (IV) : 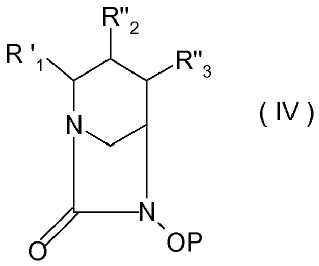 dans laquelle R'₁ et P sont tels que définis plus haut et R''₂ et R''₃ forment ensemble un hétérocycle azoté à caractère aromatique à 5 sommets renfermant 1, 2 ou 3 atomes d'azote, substitué sur un atome d'azote par une chaîne de formule -C(O)-NH-(CH₂)ₚ-(CHR'ₐ)_{q}R"' dans laquelle R'ₐ, R"', p et q sont tels que définis plus haut,
puis déprotège le radical hydroxy et soumet le composé obtenu à une réaction de sulfatation par action de SO₃ complexé, ou à l'action d'un réactif de formule Hal-CHF-CO₂alc ou de formule Hal-CF₂-CO₂alc, dans laquelle Hal représente un atome d'halogène différent du fluor et alc représente un radical alcoyle renfermant de 1 à 6 atomes de carbone, en présence d'une base, puis à une hydrolyse de l'ester d'alcoyle ainsi obtenu,
puis, le cas échéant, soumet le composé obtenu à une ou plusieurs des réactions suivantes, dans un ordre approprié :
- déprotection de la ou des fonctions aminées et, le cas échéant carboxy, présentes,
- salification,
- échange d'ions,
- dédoublement ou séparation de diastéréoisomères.

10. Procédé de préparation de composés de formule (I), telle que définie à la revendication 1, **caractérisé en ce que** l'on traite un composé de formule (II) telle que définie à la revendication 9, par une base, puis par un réactif de formule (V) :
Hal-SO₂-NH-(CH₂)ₚ-(CHR'ₐ)_{q}R''' (V)
dans laquelle Hal représente un atome d'halogène et R'ₐ, R"', p et q sont tels que définis à la revendication 9, pour obtenir un composé de formule (IVa) : 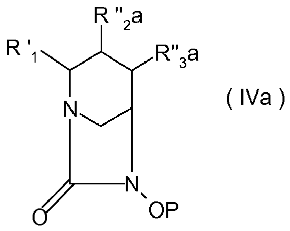 dans laquelle R'₁ et P sont tels que définis à la revendication 8 et R''₂ₐ et R''₃ₐ forment ensemble un hétérocycle azoté à caractère aromatique à 5 sommets renfermant 1, 2 ou 3 atomes d'azote, substitué sur un atome d'azote par une chaîne de formule -SO₂-NH-CH₂)ₚ-(CHR'ₐ)_{q}R"' dans laquelle R'ₐ, R"', p et q sont tels que définis plus haut,
puis déprotège le radical hydroxy et poursuit la synthèse comme décrit à la revendication 9.

11. Procédé de préparation de composés de formule (I), telle que définie à la revendication 1, **caractérisé en ce que** l'on traite un composé de formule (II) telle que définie à la revendication 9, le cas échéant en présence d'une base, par un réactif de formule (VI) :
B-C(O)-(NH)ₒ-(CH₂)ₚ-(CHR'ₐ)_{q}R"' (VI)
dans laquelle B représente un radical OH ou un atome d'halogène et R'ₐ, R"', o, p et q sont tels que définis à la revendication 9 pour obtenir un composé de formule (IVb) : 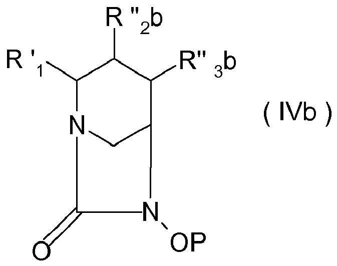 dans laquelle R'₁ et P sont tels que définis plus haut et R''_{2b} et R''_{3b} forment ensemble un hétérocycle azoté à caractère aromatique à 5 sommets renfermant 1, 2 ou 3 atomes d'azote, substitué sur un atome d'azote par une chaîne de formule -C(O)-(NH)ₒ-(CH₂)ₚ-(CHR'ₐ)_{q}R"' dans laquelle R'ₐ, R"', o, p et q sont tels que définis plus haut, puis poursuit la synthèse comme décrit à la revendication 9.

12. Procédé de préparation de composés de formule (I), telle que définie à la revendication 1, **caractérisé en ce que** l'on traite un composé de formule (II) telle que définie à la revendication 9, en présence d'une base, par un réactif de formule (VII) :
S=C(NHP')₂ (VII)
dans laquelle P' représente un groupement protecteur de la fonction amino, pour obtenir un composé de formule (IVc) : 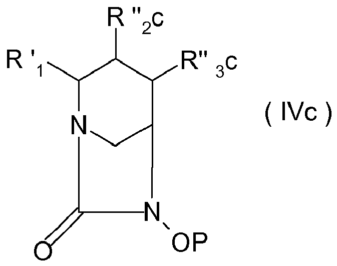 dans laquelle R'₁ et P sont tels que définis à la revendication 9 et R''_{2c} et R''_{3c} forment ensemble un hétérocycle azoté à caractère aromatique à 5 sommets renfermant 1, 2 ou 3 atomes d'azote, substitué sur un atome d'azote par une chaîne de formule -C(=NH)-NHP' dans laquelle P' est tel que défini plus haut,
puis poursuit la synthèse comme décrit à la revendication 9.

13. Procédé de préparation de composés de formule (I), telle que définie à la revendication 1, **caractérisé en ce que** l'on traite un composé de formule (II) telle que définie à la revendication 9, par un réactif de formule (VIII) :
O=C=N-(CH₂)ₚ-(CHR'ₐ)_{q}R''' (VIII)
dans laquelle R'ₐ, R"', p et q sont tels que définis à la revendication 9, pour obtenir un composé de formule (IVd) : 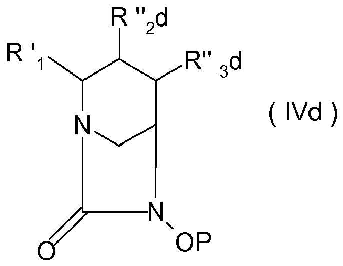 dans laquelle R'₁ et P sont tels que définis à la revendication 9 et R''_{2d} et R''_{3d} forment ensemble un hétérocycle azoté à caractère aromatique à 5 sommets renfermant 1, 2 ou 3 atomes d'azote, substitué sur un atome d'azote par une chaîne de formule -(CO)-NH-(CH₂)ₚ-(CHR'ₐ)_{q}R''' dans laquelle R'ₐ, R"', p et q sont tels que définis plus haut, puis poursuit la synthèse comme décrit à la revendication 9.

14. Procédé de préparation de composés de formule (I), telle que définie à la revendication 1, **caractérisé en ce que** l'on traite un composé de formule (II') : 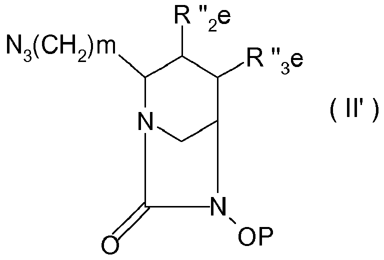 dans laquelle R''₂ₑ et R''₃ₑ forment ensemble un hétérocycle azoté à caractère aromatique à 5 sommets renfermant 1, 2 ou 3 atomes d'azote éventuellement substitué par un radical (C₁-C₆)alc m est tel que défini à la revendication 1 et P est tel que défini à la revendication 9, par un réactif de formule (IX) :
CH₃-S-C(=NP')NHP' (IX)
dans laquelle P' est tel que défini à la revendication 9, pour obtenir un composé de formule (X) : 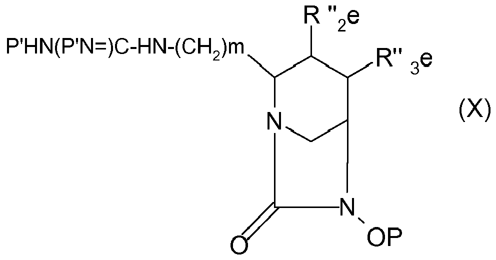 dans laquelle R''₂ₑ, R''₃ₑ, m, P et P' sont tels que définis plus haut,
puis poursuit la synthèse comme décrit à la revendication 9.

15. Procédé de préparation de composés de formule (I), telle que définie à la revendication 1, dans laquelle R₄ représente un groupe CO-NH₂ ou CO-NH(C₁-C₆)alc, **caractérisé en ce que** l'on traite un composé de formule (II) telle que définie à la revendication 9, avec le triméthylsilyl isocyanate ou avec un isocyanate de formule (C₁-C₆)alc-N=C=O, pour obtenir un composé correspondant de formule (IV), puis poursuit la synthèse comme décrit à la revendication 9.

16. Les composés de formules (IV), (IVa), (IVb), (IVc), (IVd) et (X) telles que définies aux revendications 9, 10, 11, 12, 13 et 14.

17. A titre de médicaments, les composés tels que définis à l'une quelconque des revendications 1 à 7, ainsi que leurs sels avec les acides et les bases pharmaceutiquement acceptables.

18. A titre de médicaments, les composés tels que définis à la revendication 8.

19. Compositions pharmaceutiques contenant, à titre de principe actif, au moins un médicament selon l'une des revendications 17 et 18.
